# EUROPEAN PATENT APPLICATION

(11) **EP 4 628 868 A1**
(43) Date of publication of application: **08.10.2025**
(21) Application number: 23897337.4
(22) Date of filing: 27.10.2023
(51) Int. Cl.: G01N 1/42, G01N 21/64, C12M 1/00, G02B 21/00, G02B 21/28, G02B 21/30, G02B 21/34

(54) **FREEZING DEVICE, MICROSCOPE, FREEZING METHOD, AND OBSERVATION METHOD**

(30) Priority: 29.11.2022 JP 2022189880; 27.07.2023 JP 2023122175
(71) Applicant: The University of Osaka, Suita-shi, Osaka 565-0871 (JP)
(72) Inventor: FUJITA, Katsumasa, Suita-shi, Osaka 565-0871 (JP); YAMANAKA, Masahito, Suita-shi, Osaka 565-0871 (JP); KUMAMOTO, Yasuaki, Suita-shi, Osaka 565-0871 (JP); TSUJI, Kosuke, Suita-shi, Osaka 565-0871 (JP)
(74) Representative: Banse & Steglich Patentanwälte PartmbB
(86) International application number: PCT/JP2023/038854
(87) International publication number: WO 2024/116681

(57) **Abstract**

A freezing apparatus according to the present embodiment includes: a sample holder (400) configured to hold a sample (S) in a state where a volume of a liquid in the sample (S) in a cryogen contact area differs from a height of the liquid outside the cryogen contact area; and sample freezing means for freezing the sample (S) in the cryogen contact area with a cryogen.

## Description

### Technical Field

The present invention relates to a freezing apparatus, a microscope, a freezing method, and an observation method, and particularly relates to a technique for freezing a sample.

### Background Art

In observing a biological sample with an optical microscope, the sample is sometimes fixed in advance to avoid changes in the sample during observation or by observation pretreatment. In this case, the fixation is performed through use of chemical fixation with aldehydes that crosslink proteins or dehydration fixation with organic solvents. These fixing methods are sufficient to maintain morphological information at the level of optical microscopic observation. However, some molecules may be oxidized by aldehydes or dissolved in organic solvents. Furthermore, the fixation reaction takes minutes, and the fixation reaction in a deep portion takes more time if the sample is thick. Additionally, there is also a problem in which not all molecules are fixed.

### Citation List

### Non Patent Literature

Non Patent Literature 1: Fuest, et al., J. Microscopy. 272, 87 (2018).
Non Patent Literature 2: Huebinger, et al., Science Advanced 7, eabk0882 (2021)

### Summary of Invention

### Technical Problem

In the background described above, cryofixation has recently begun to attract attention as a new method of fixing biological samples. Cryofixation is a method of physically stopping the movement of molecules and ions in a sample by freezing water. If freezing is carried out in a short time, biological samples can be fixed in their original state. Accordingly, cells can be observed and preserved as they are.

Non Patent Literature 1 discloses a method using a heat sink and a heater. In this method, a thin NiCr heater is placed on a heat sink cooled by liquid nitrogen. The sample placed on the heater is frozen at an optional timing by controlling the heater on and off. The sample is placed in the micro flow path to reduce the sample volume. This method has successfully frozen nematodes within several tens of msec. However, with this related art, the freezing speed is restricted by the thickness of the heater.

Non Patent Literature 2 discloses a method of freezing a sample using a diamond substrate. In this method, the diamond substrate is pressed against the sample and the diamond substrate is cooled with liquid nitrogen. Furthermore, nitrogen gas produced on the diamond substrate is exhausted. This method is constrained with respect to sample manipulation (such as chemical loading) and maintenance of the sample ambient environment (such as ion concentration) during microscopic observation. In addition, it is unclear as to how quickly a sample can be frozen.

The present disclosure has been made in consideration of the points described above and an object thereof is to provide a freezing apparatus, a microscope, a freezing method, and an observation method that enable a sample to be frozen in an appropriate manner.

### Solution to Problem

A freezing apparatus according to the present embodiment includes: a sample holder configured to hold a sample containing a liquid in a state where a volume of the liquid in a cryogen contact area differs from a volume of the liquid outside the cryogen contact area; and sample freezing means for freezing the sample in the cryogen contact area with a cryogen.

In the freezing apparatus described above, the sample holder may hold the sample in a state where a height of a liquid level of the sample in the cryogen contact area is lower than outside the cryogen contact area.

The freezing apparatus described above may include an adjustment mechanism configured to adjust a height of a liquid level of the liquid.

In the freezing apparatus described above, the sample holder may be provided with a through-hole, and the height of the liquid level of the liquid may be adjusted by sucking the liquid from the through-hole.

In the freezing apparatus described above, a space enclosed by the sample holder may include: a first supply port to which a chemical fixative for chemically fixing the sample is supplied; and a second supply port to which a staining dye for staining the sample or a decolorizing agent for decolorizing the stained sample is supplied.

In the freezing apparatus described above, the sample holder may be provided with a sample retainer configured to press the sample, in which the sample retainer may include an opening portion that corresponds to the cryogen contact area.

In the freezing apparatus described above, the height of the liquid level of the liquid may be adjusted by pressing a pressing member provided above the sample against the sample.

The freezing apparatus described above may further include a low-temperature maintaining mechanism configured to maintain the cryogen at a low temperature after freezing the sample.

The freezing apparatus described above may further include a measurement mechanism configured to measure the height of the liquid level of the liquid.

In the freezing apparatus described above, after freezing the sample, the sample may be preserved in the frozen state.

In the freezing apparatus described above, the sample preserved in the frozen state may be thawed and cultured.

A microscope according to the present embodiment includes: the freezing apparatus described above; and an objective lens configured to receive light from the sample, in which the cryogen contact area corresponds to a field of view of the objective lens, and the sample freezing means may freeze a sample in the field of view of the objective lens.

The microscope described above may further include a cooling block provided directly above the sample, in which a cooling tube through which a liquid for cooling the cooling block circulates is provided inside the cooling block, the cooling block is provided with a through-hole through which a cryogen for freezing the sample passes, and the cooling block may be provided with at least one of a temperature sensor and a heater.

A freezing method according to the present embodiment includes the steps of: holding a sample containing a liquid with a sample holder in a state where a volume of the liquid in a cryogen contact area differs from a volume of the liquid outside the cryogen contact area; and freezing the sample in the cryogen contact area with a cryogen.

An observation method according to the present embodiment includes the steps of: freezing the sample in a field of view of an objective lens using the freezing method described above; and observing the frozen sample in the cryogen contact area with the objective lens.

In the observation method described above, the sample may be observed while the sample is frozen.

### Advantageous Effects of Invention

According to the present disclosure, a freezing apparatus, a microscope, a freezing method, and an observation method that enable a sample to be frozen in an appropriate manner can be provided.

### Brief Description of Drawings

Fig. 1 is an XZ cross-sectional view schematically showing an overall configuration of a microscope using a sample holder according to a first embodiment.
Fig. 2 is an XZ cross-sectional view showing a configuration of the sample holder in an enlarged manner.
Fig. 3 is a perspective view showing a configuration of a sample placement unit according to an example.
Fig. 4 is an exploded perspective view showing the configuration of the sample placement unit according to the example.
Fig. 5 is an XZ cross-sectional view showing the configuration of the sample placement unit according to the example.
Fig. 6 is an XZ cross-sectional view showing a state where a metal member for maintaining cooling of a cryogen has been inserted.
Fig. 7 is a diagram showing tomographic images captured at different heights of a liquid level.
Fig. 8 is an XZ cross-sectional view showing a configuration provided with a pressing member to be pressed against a sample.
Fig. 9 is a schematic view showing a configuration of a microscope according to a second embodiment.
Fig. 10 is an XZ cross-sectional view schematically showing a configuration of a microscope according to a third embodiment.
Fig. 11 is a schematic view showing a configuration of a sample holder of the microscope according to the third embodiment.
Fig. 12 is an XZ cross-sectional view schematically showing a configuration according to a modification of the third embodiment.
Fig. 13 is a diagram for illustrating a sample substrate and a freezing method according to a fourth embodiment.
Fig. 14 is a diagram for illustrating a method of thawing a sample substrate.
Fig. 15 is a diagram for illustrating a method of observing a frozen sample.
Fig. 16 is a diagram for illustrating a method of observing a thawed sample and a refrozen sample.
Fig. 17 is a diagram showing a configuration of a sample substrate including a reservoir.
Fig. 18 is an XZ cross-sectional view showing a configuration of a sample holder that holds the sample substrate.
Fig. 19 is a cross-sectional view for illustrating a method of adjusting a liquid volume on the sample substrate.
Fig. 20 is a cross-sectional view for illustrating a method of adjusting a liquid volume on the sample substrate.
Fig. 21 is a cross-sectional view schematically showing a configuration of the sample holder.
Fig. 22 is an exploded perspective view showing a configuration of a sample holder including a cooling block.
Fig. 23 is a cross-sectional view showing the configuration of the sample holder including the cooling block.

### Description of Embodiments

Embodiments to which the present invention is applicable will be described below. The following description explains embodiments of the present invention, and the present invention is not limited to the following embodiments. For clarity of explanation, the following description has been omitted and simplified as appropriate. Moreover, those skilled in the art will be able to easily change, add, and convert each element of the following embodiments within the scope of the present invention. Note that the same reference numerals and characters in each drawing indicate similar elements, and the description thereof will be omitted as appropriate.

### First Embodiment

A freezing apparatus, a freezing method, a microscope, and an observation method according to the present embodiment will be described. While the freezing apparatus is described as being mounted on a microscope in the following examples, the freezing apparatus need not be mounted on a microscope. In other words, the freezing apparatus and the freezing method can be used in applications other than observations with a microscope. For example, a sample can be frozen and preserved by a freezing method using the freezing apparatus.

In addition, a sample preserved in the frozen state may be thawed and cultured. In other words, after thawing the sample, a culture apparatus or the like may culture cells or the like in the sample. The cultured sample can be used in regenerative medicine, reproductive medicine, cellular medicine, drug discovery research, animal husbandry, food production, and the like.

Using the microscope according to the present embodiment enables a sample in a frozen state to be observed. The sample can be fixed in a frozen state. Therefore, an exposure time of a photodetector can be increased and the sample can be observed with a high signal-to-noise ratio. A sample labeled with a fluorescent substance may be observed under a fluorescence microscope. Alternatively, an unlabeled sample may be observed under a microscope.

An optical microscope (hereinafter also simply referred to as a microscope) according to the present embodiment freezes a sample with a cryogen. The microscope includes sample freezing means in which the cryogen is supplied. The sample freezing means supplies the cryogen to the sample in the field of view of the objective lens. The cryogen supplied from the sample freezing means freezes the sample. The sample in the field of view of the objective lens can be frozen. Accordingly, a sample being frozen or a frozen sample can be easily observed.

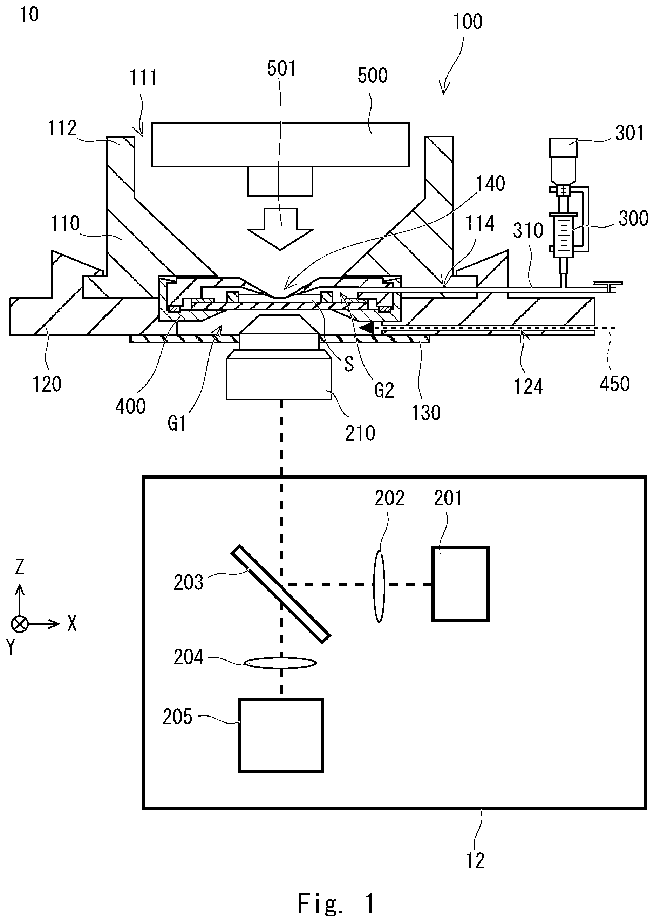

A configuration of a microscope including the sample freezing means will be described with reference to Fig. 1. Fig. 1 is a diagram showing an overall configuration of a microscope. Note that, in the following drawings, an XYZ three-dimensional orthogonal coordinate system is appropriately shown. The Z direction is a direction parallel to the optical axis. The XY plane is a plane orthogonal to the Z direction, and serves as a focal plane on which the sample is arranged.

A microscope 10 includes a microscope main body 12, a sample placement unit 100, an objective lens 210, a syringe 300, and a cryogen feeding apparatus 500. Since the microscope 10 is an inverted microscope, the objective lens 210 is arranged below a sample S. The objective lens 210 may include a mechanism that restores a position of a focal plane of the objective lens to an observation position of the sample when the position of the focal plane deviates from the observation position in the Z direction. For example, the mechanism uses a piezo-stage for an objective lens. Obviously, the microscope 10 may be an upright microscope. In the case of an upright microscope, the objective lens 210 is installed above the sample S. Note that the sample S contains a liquid.

The sample placement unit 100 includes a first plate 110, a second plate 120, and a sample holder 400. The sample holder 400 holds the sample S. A configuration of the sample holder 400 will be described later. The sample holder 400 is placed on the second plate 120. Furthermore, the first plate 110 is arranged on the second plate 120. The first plate 110 is attachable to and detachable from the second plate 120. The second plate 120 may include a mechanism for fixing the first plate 110. In addition, the second plate 120 is fixed to a stage of the microscope or the like via a plate for fixing the second plate to the stage of the microscope or the like.

The second plate 120 is arranged so as to cover a peripheral edge portion of the sample holder 400. The sample holder 400 is held between the second plate 120 and the first plate 110. The second plate 120 and the first plate 110 are formed in hollow shapes. Hollow portions of the first plate 110 and the second plate 120 constitute a space for arranging the objective lens 210 and the sample holder 400.

The first plate 110 includes a cylindrical portion 112 that extends upward. A hollow space of the cylindrical portion 112 constitutes a cryogen storage tub 111 for storing a cryogen. A liquid cryogen 501 is fed to the cryogen storage tub 111 from an cryogen feeding apparatus 500. The cryogen 501 is an organic solvent and can be, for example, a liquid propane/isopentane mixed solution. Alternatively, the cryogen 501 may be liquid ethane, liquid propane, or the like. Furthermore, the cryogen 501 may be a solid.

The cryogen feeding apparatus 500 causes the cryogen 501 to drip into the cryogen storage tub 111 from above. The cryogen 501 fed into the cryogen storage tub 111 comes into contact with the sample S in a field of view 140 of the objective lens 210. Accordingly, the sample S in the field of view 140 is cooled and frozen. The cryogen 501 is in contact with the sample S, and by storing the cryogen 501 in the cryogen storage tub 111, the sample S can be cooled down to the temperature of the cryogen 501 in an efficient manner. The first plate 110 may be provided with a discharge port (not illustrated) of the cryogen 501 and the cryogen 501 stored in the cryogen storage tub 111 may be removed. Accordingly, the cryogen feeding apparatus 500 can continuously supply the cryogen 501.

In this manner, the cryogen storage tub 111, the cryogen feeding apparatus 500 and the like constitute a sample freezing mechanism that freezes the sample S with the cryogen 501. In the cryogen feeding apparatus 500, a high-speed electromagnetic valve and the like are provided in a flow path of the liquid cryogen 501. The sample S can be frozen at a desired timing by controlling opening and closing of the valve. Alternatively, the opening and closing of the electromagnetic valve may be controlled by a signal from the sample S. Yet alternatively, a user may manually supply the cryogen 501.

The objective lens 210 is arranged directly below the sample S. The hollow portion provided in the second plate 120 constitutes an objective lens space G1 in which the objective lens 210 is to be arranged. Furthermore, the microscope main body 12 is arranged below the objective lens 210. An optical system for propagating illumination light and observation light is arranged in the microscope main body 12. For example, the microscope main body 12 includes a light source 201, a lens 202, a beam splitter 203, an imaging lens 204, and a photodetector 205.

The light source 201 generates light for illuminating the sample S. The illumination light from the light source 201 is refracted by the lens 202 and is incident to the beam splitter 203. The beam splitter 203 is a one-way mirror or the like and transmits approximately half of the incident light and reflects approximately half of the incident light. The beam splitter 203 reflects a part of the illumination light toward the objective lens 210. The objective lens 210 concentrates the illumination light onto the sample S. Accordingly, the sample S can be illuminated.

The objective lens 210 receives light from the sample S. The light from the sample S is refracted by the objective lens 210 and is incident to the beam splitter 203. The light transmitted through the beam splitter 203 is incident to the photodetector 205 via the imaging lens 204. The imaging lens 204 forms an image of the sample S on the photodetector 205. The photodetector 205 is a CCD camera or a CMOS sensor and includes a plurality of pixels arranged in a two-dimensional array. Therefore, the photodetector 205 can capture an enlarged image of the sample S. Note that the optical system of the microscope main body 12 is not limited to the illustrated optical system. For example, an optical filter such as a wavelength filter may be arranged in the optical system.

The syringe 300 serves as an adjustment mechanism for adjusting a height of a liquid level of the sample S. The syringe 300 is provided with a micrometer 301 and a screw mechanism. The syringe 300 is connected to a through-hole 114 of the first plate 110 via a piping tube 310. The through-hole 114 is connected to a sample space G2 inside the sample holder 400. The syringe 300 can adjust pressure in the sample space G2 around the sample S. In other words, by operating the syringe 300 with the micrometer 301, a gas in the sample space G2 can be sucked or a gas can be supplied to the sample space G2. Accordingly, air pressure in the sample space G2 in which the sample S is present can be adjusted. Therefore, since air pressure difference between the sample space G2 inside the sample holder 400 and a space outside the sample holder 400 is created, the height of the liquid level of the sample S can be adjusted in the field of view 140 of the objective lens 210. Note that methods other than gas suction and gas supply can be used to adjust the height of the liquid level. For example, a sample retainer to be described later may be used or an absorbent material that absorbs liquids may be used. Bringing the absorbent material into contact with the liquid lowers the liquid level.

The syringe 300 with the micrometer 301 may constitute a mechanism that is electrically powered and controlled by an external signal. In this case, timings of height adjustment of the liquid level by the syringe 300 with the micrometer 301 and feeding of the cryogen by the cryogen feeding apparatus 500 can also be controlled.

In addition, the sample placement unit 100 may include a measurement mechanism that measures the height of the liquid level in the field of view 140. For example, the measurement mechanism that measures the height of the liquid level detects reflected light from a liquid surface using the objective lens 210 and the photodetector 205. Alternatively, the measurement mechanism may measure the height of the liquid level from an upper portion of the field of view using a laser surveying instrument or the like. The measurement mechanism may be a mechanism in which an electrode pair is placed across a field of view and a liquid volume is estimated from a value of a current flowing through the liquid. The measurement mechanism can be used for timing control of height adjustment and freeze start.

A through-hole 124 connected to the objective lens space G1 in which the objective lens 210 is arranged is formed in the second plate 120. The through-hole 124 is formed in the X direction. The through-hole 124 penetrates from an outer circumferential surface to an inner circumferential surface of the second plate 120. An inert gas 450 is supplied to the through-hole 124 in order to prevent dew condensation on the objective lens 210. The inert gas 450 is, for example, dried nitrogen gas. The inert gas 450 is supplied to the objective lens space G1 via the through-hole 124.

In this case, a rubber 130 is provided around the objective lens 210. The rubber 130 is, for example, a sheet formed of a silicone resin or the like. In addition, the rubber 130 is provided with a hole into which the objective lens 210 is to be inserted. The objective lens 210 is fitted into the rubber 130. The objective lens space G1 surrounded by the rubber 130, the sample holder 400, the objective lens 210, and the second plate 120 becomes a nitrogen atmosphere. Accordingly, dew condensation on the objective lens 210 can be prevented. Although not illustrated, the second plate 120 may be provided with a discharge port of nitrogen gas.

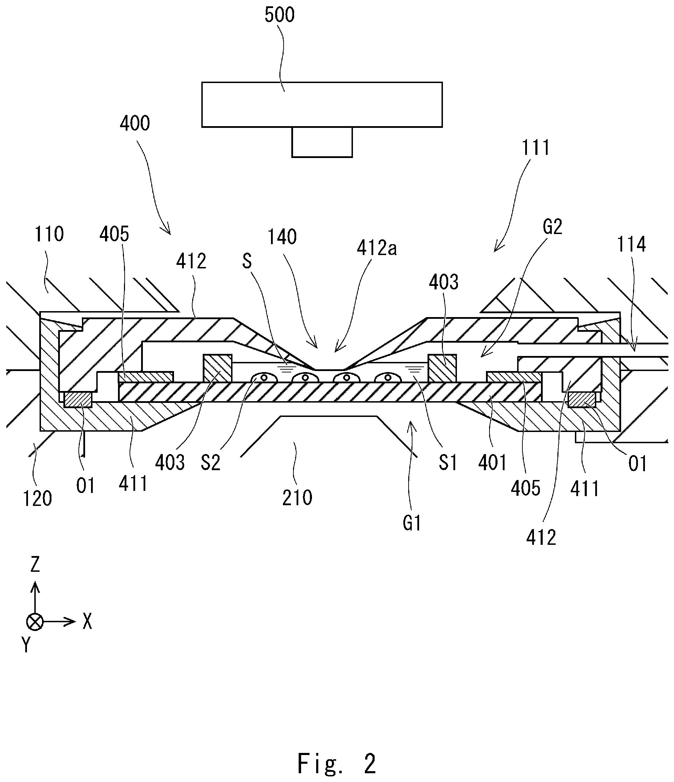

A configuration of the sample holder 400 will now be described with reference to Fig. 2. Fig. 2 is an XZ cross-sectional view showing the sample holder 400 and a periphery thereof in an enlarged manner. The sample holder 400 includes a substrate 401, a wall 403, a spacer 405, a base 411, a sample retainer 412, and an O-ring O1.

The substrate 401 is a plate formed of a transparent material. More specifically, for example, the substrate 401 is a cover glass formed of soda glass, quartz, or the like. The sample S is arranged on the substrate 401. The sample S contains a liquid S1 and a biological sample S2. The liquid S1 is a buffer solution, a culture medium, or the like. The biological sample S2 is cultured cells or the like and is arranged in the liquid S1. Since the biological sample S2 is immersed in the liquid S1, the biological sample S2 can be prevented from drying out. The illumination light from the objective lens 210 passes through the substrate 401 and illuminates the sample S. In addition, observation light from the sample S passes through the substrate 401 and is incident to the objective lens 210.

The wall 403 is arranged on the substrate 401. The wall 403 is formed in a closed ring shape on an XY plane. Therefore, the wall 403 forms a liquid storage tub for holding the sample S that contains the liquid S1. Since the periphery of the sample S is surrounded by the wall 403, the liquid S1 is dammed by the wall 403. Therefore, the sample S is held in a state where the liquid level of the liquid S1 has reached a predetermined height. The wall 403 is formed of a material such as silicone rubber which does not absorb water. The wall 403 may be bonded and fixed to the substrate 401.

The substrate 401 is arranged on a base 411 that includes an opening. Therefore, a peripheral edge portion of the substrate 401 is placed on the base 411. The sample retainer 412 is arranged on the peripheral edge portion of the base 411. The base 411 serves as a lower holder and the sample retainer 412 serves as an upper holder. The sample retainer 412 is arranged on the base 411 via the spacer 405. In addition, the O-ring O1 for sealing the sample space G2 is arranged between the sample retainer 412 and the base 411. Note that a member for sealing the sample space G2 is not limited to an O-ring and rubber, metal, a liquid, or the like can be used.

The sample retainer 412 extends from outside the wall 403 to the top of the sample S. The sample retainer 412 includes an opening portion 412a. A distance between a tip of the sample retainer 412 and the substrate 401 in the opening portion 412a can be adjusted by the spacer 405. For example, the distance between the tip of the sample retainer 412 and the substrate 401 is around 20 to 100 µm. The opening portion 412a is arranged on an optical axis of the objective lens 210 or, in other words, in the field of view 140 of the objective lens 210. In an XY plan view, the opening portion 412a has a circular shape of, for example, around several mm in diameter so as to correspond to the field of view 140. In addition, the opening portion 412a may have a square shape or a rectangular shape. A size of the opening portion 412a is also not particularly limited. Increasing the opening portion 412a enables a large amount of cells to be simultaneously frozen. Accordingly, samples can be appropriately frozen and preserved.

The sample retainer 412 is in contact with the liquid S1 outside of the field of view 140 of the objective lens 210. A difference in the height of the liquid level of the liquid S1 is created between the outside and the inside of the opening portion 412a. Specifically, the height of the liquid level inside the opening portion 412a is lower than the height of the liquid level outside the opening portion 412a. The height of the liquid level in the field of view 140 of the objective lens 210 can be made lower than the height of the liquid level outside of the field of view 140. Alternatively, the state of the inside and the outside of the opening portion 412a is not limited to a state where a difference in the height of the liquid level is created between the outside and the inside. For example, in a state where a liquid storage volume of the outside of the opening portion 412a is larger than that of the inside, the height of the liquid level outside of the opening portion 412a need not be higher than that of the inside.

As described above, the sample holder 400 can be used for applications other than microscopy applications. For example, the sample holder 400 and the cryogen feeding apparatus 500 can be applied to a freezing apparatus for freezing samples. The sample holder 400 may hold a sample in a state where a volume of liquid in a cryogen contact area in which the sample is frozen differs from a volume of liquid in an area outside the cryogen contact area. The cryogen feeding apparatus 500 freezes the sample S in the cryogen contact area using the cryogen 501. For example, accordingly, the sample can be appropriately preserved. In the case of a microscopy application, the field of view of the objective lens 210 corresponds to the cryogen contact area. For example, the cryogen contact area can also be described as an area where the cryogen comes into direct contact with the sample. The cryogen contact area may be adjusted or changed by adjusting or changing a contact range of the cryogen. The sample S freezes in the cryogen contact area. Furthermore, the sample S may also freeze outside the cryogen contact area. The cryogen passes through the opening portion 412a and comes into contact with the sample. Therefore, the cryogen contact area can also be described as an area that corresponds to the opening portion 412a to which the cryogen is supplied.

The user can observe the frozen sample S. The cryogen feeding apparatus 500 may be configured to feed the cryogen 501 during observation of the sample S. Accordingly, the user can observe the sample S while the sample S is being frozen by the cryogen 501. Therefore, the user can observe the gradual freezing of the sample S.

In addition, the user may adjust a timing of freezing during observation. In other words, when an appropriate timing arrives during the observation of the sample S, the user operates the cryogen feeding apparatus 500 to feed the cryogen 501. The user can control the timing of feeding the cryogen by opening and closing a valve or the like provided in the cryogen feeding apparatus 500. Accordingly, the user can freeze the sample at a desired timing. The valve may be opened and closed by the user observing the sample by operating a switch or the like.

Alternatively, the microscope may be provided with a control unit that controls opening and closing of the valve. The control unit controls the opening and closing of the valve using various signals. Accordingly, the freeze timing can be automatically adjusted. Various signals can be used as the signal to trigger opening and closing of the valve.

For example, valve opening/closing may be controlled based on signals from the sample S. Specifically, the control unit outputs control signals based on optical signals and electrical signals from the sample. The intensities of scattered light or the intensities of fluorescence can be used as the optical signals from the sample S. Furthermore, an image of the sample S may be captured and the valve may be opened or closed according to a form of the sample S such as a shape or a size thereof. For example, when the sample S is a pulsating internal organ, the control unit outputs control signals in response to changes in the form of the sample S. Accordingly, the sample S can be frozen in conjunction with the pulsation timings.

Alternatively, if optical stimulation, electrical stimulation, electromagnetic stimulation, magnetic stimulation, or the like is applied to the sample S based on signals for stimulating the sample S, the control unit opens and closes the valve in response to the signals that stimulates the sample S. Accordingly, the sample S can be frozen in conjunction with the timing of stimulation. Furthermore, the stimulation of the sample S is not limited to optical stimulation, electrical stimulation, electromagnetic stimulation, and magnetic stimulation. The stimulation of the sample S can be provided by drugs, temperature, mechanical stimulation, electromagnetic waves, electron beams, radiation, optogenetics, photodissociation of caged compounds, or the like. Use of signals for stimulating the sample S allows the sample to be frozen at an appropriate timing. Controlling the opening and closing of the valve in this manner enables the timing of introducing the cryogen to be adjusted. Of course, the control unit may control the timing of introducing the cryogen using two or more signals.

The sample retainer 412, the base 411, the spacer 405, and the like are formed of a material with little expansion and contraction due to temperature change. In other words, the sample retainer 412, the base 411, the spacer 405, and the like are formed of a material with a low coefficient of thermal expansion. For example, the sample retainer 412, the base 411, and the spacer 405 can be formed of stainless steel, an Invar alloy, a super Invar alloy, or the like.

The first plate 110 is provided with the through-hole 114. As shown in Fig. 1, the sample space G2 and the syringe 300 are connected via the through-hole 114 of the first plate 110. Accordingly, the height of the liquid level in the field of view 140 can be adjusted.

For example, when the syringe 300 supplies a gas to the sample space G2, pressure inside the sample space G2 increases. As a result, the liquid level in the field of view 140 rises. In addition, when the syringe 300 sucks the air inside the sample space G2, pressure inside the sample space G2 decreases. As a result, the liquid level in the field of view 140 falls. Since air pressure in the sample space G2 becomes lower than air pressure in the cryogen storage tub 111, the liquid level falls in the field of view 140. Accordingly, the user can adjust the height of the liquid level of the liquid S1 in the field of view 140. In other words, the user can adjust the liquid volume in the field of view 140. The user freezes and observes the sample S in a state where the height of the liquid level in the field of view 140 is set to around 20 to 100 µm.

In this manner, the syringe 300 and the like can be used as a liquid volume adjustment mechanism. The sample can be frozen and fixed with ease and high reproducibility. Since the height of the liquid level can be made approximately constant, observation with high reproducibility can be achieved. Obviously, a suction mechanism other than the syringe 300 may be used to make the sample space G2 a negative pressure space. In addition, any method other than a suction mechanism may be used as long as the method adjusts the height of the liquid level using a pressure difference.

A sufficient volume of the liquid S1 is secured in the liquid storage tub formed by the wall 403. For example, the liquid storage tub formed by the wall 403 is larger than the opening portion 412a. The biological sample S2 is configured to be less likely to dry out even when the liquid volume in the field of view 140 is reduced. In other words, even when the height of the liquid level in the field of view 140 is set to around 20 to 100 µm, the liquid level outside the field of view 140 is higher than inside the field of view 140. After adjustment of the liquid volume in the field of view 140, the cryogen feeding apparatus 500 feeds the liquid cryogen 501 at any timing while microscopic observation is being performed. The temperature of the sample S drops as soon as the cryogen 501 comes into contact with the sample S and the sample S is frozen immediately. Since the height of the liquid level in the field of view 140 is set to around 20 to 100 µm, the sample S can be frozen in a short period of time of around several msec.

Furthermore, the cryogen storage tub 111 reaches a surface of the sample S. Therefore, chemicals and the like can also be fed into the sample S from the cryogen storage tub 111 until just before the cryogen feed. Since liquids such as a reagent can be supplied from the cryogen storage tub 111 in a timely manner, the periphery of the sample can be maintained in an optimal environment for the sample S.

In addition, the microscope 10 may include a low-temperature maintaining mechanism that maintains the cryogen at a low temperature after freezing the sample. For example, the temperature of the sample S can also be maintained at a low temperature by placing a metal member at the temperature of liquid nitrogen in the cryogen storage tub 111 after feeding the cryogen 501. Specifically, a metal rod cooled to -180°C to -190°C with liquid nitrogen is brought into contact with the cryogen 501. Accordingly, since the cryogen 501 is maintained at a low temperature, freezing of the sample S can be maintained even when observation continues for a long period of time. In addition, a low temperature may be maintained by cooling the sample holder 400 from outside.

Furthermore, the microscope 10 may include a replacement apparatus that replaces the cryogen 501 with liquid nitrogen after the sample S is frozen. For example, a discharge path of the cryogen 501 is provided in the cryogen storage tub 111. After freezing with the cryogen 501, liquid nitrogen is supplied to the cryogen storage tub 111 and, at the same time, the cryogen 501 is discharged via the discharge path. Accordingly, the cryogen 501 can be replaced with liquid nitrogen. Even if background light is generated by the cryogen, an effect of the background light on optical measurements can be suppressed. When optical measurements are affected by the cryogen 501 that is an organic solvent, replacing the cryogen 501 in the cryogen storage tub 111 with liquid nitrogen enables more appropriate observations to be performed.

In addition, the sample placement unit 100 may include a mechanism that enables, after freezing the sample S, the frozen sample S to be transferred to a cryopreservation apparatus in a frozen state. For example, the frozen sample S is transferred to a container filled with liquid nitrogen and the container is transferred into an apparatus capable of cryopreservation at the liquid nitrogen temperature. Accordingly, cryopreservation of the sample and observation of the sample by other methods of electron microscopic observation can be performed.

### Example

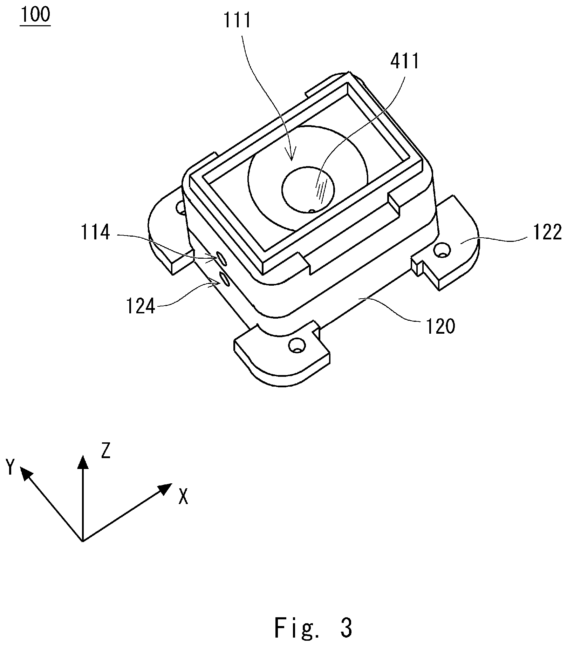

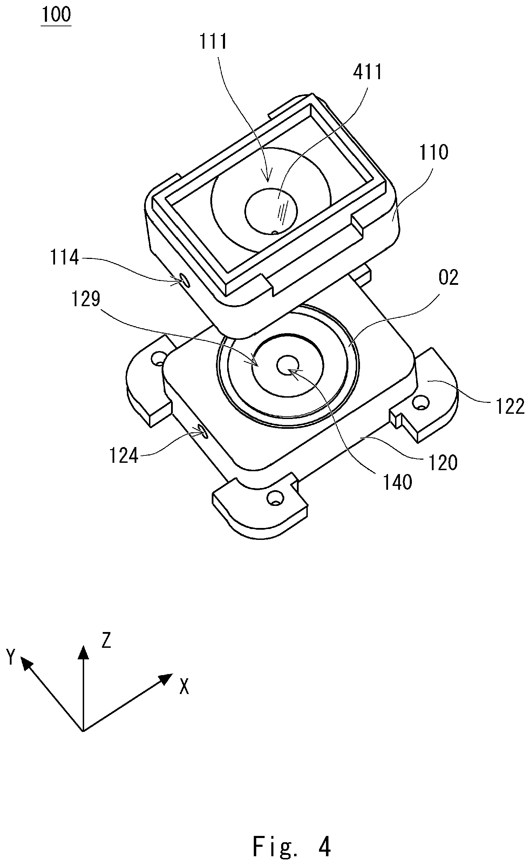

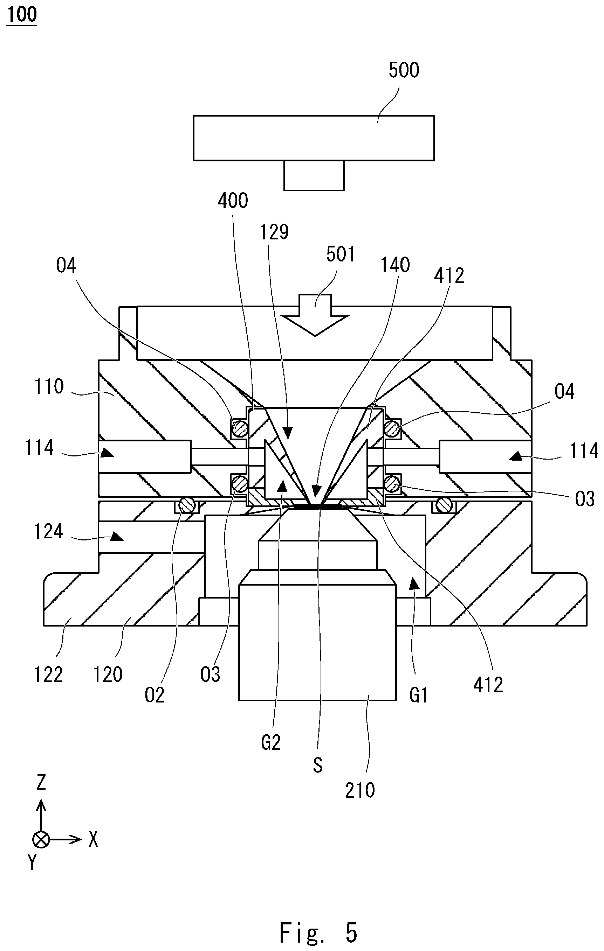

Next, a device example of the sample placement unit 100 will be described with reference to Figs. 3 to 5. Fig. 3 is a perspective view showing the sample placement unit 100 and Fig. 4 is an exploded perspective view of the sample placement unit 100. Fig. 5 is a cross-sectional view showing a configuration of the sample placement unit 100.

As shown in Figs. 3 and 4, the first plate 110 is arranged on the second plate 120. An O-ring O2 is arranged between the first plate 110 and the second plate 120. The through-hole 124 is formed in a side surface of the second plate 120. As described earlier, nitrogen gas or the like for preventing dew condensation on the objective lens 210 is supplied to the through-hole 124. The second plate 120 is provided with an adapter 122. For example, the adapter 122 includes a bolt hole and is fixed to a stage of the microscope with a bolt or the like.

The cryogen storage tub 111 is formed in an upper surface of the first plate 110. The cryogen storage tub 111 is formed in a conical shape and reaches the field of view 140 of the objective lens 210. The through-hole 114 is formed in a side surface of the first plate 110. As shown in Fig. 5, the through-hole 114 reaches the sample space G2. The piping tube 310 (refer to Fig. 1) is connected to the through-hole 114. Therefore, air pressure in the sample space G2 can be adjusted. As shown in Fig. 5, the through-hole 114 is provided on both sides of the first plate 110. In other words, the first plate 110 includes the through-hole 114 that extends toward a +X side from the sample space G2 and the through-hole 114 that extends toward a -X side from the sample space G2.

A space inside the O-ring O2 constitutes a storage space 129 of the sample holder 400. A storage space 129 is a circular space in an XY plan view. The sample holder 400 is held between the first plate 110 and the second plate 120 in a state where the sample holder 400 is arranged above the storage space 129. The sample S is arranged in the field of view 140 by storing the sample holder 400 in the storage space 129.

As shown in Fig. 5, O-rings O3 and O4 are provided on an outer circumferential surface of the sample holder 400. The O-ring O3 and the O-ring O4 are arranged between the first plate 110 and the sample holder 400. Specifically, the O-rings O3 and O4 are in contact with the outer circumferential surface of the sample retainer 412. Accordingly, airtightness of the sample space G2 is maintained. Therefore, adjustment of air pressure by the syringe 300 can be appropriately performed.

The sample holder 400 includes the sample retainer 412. The sample retainer 412 is arranged on the sample S. While the sample S is arranged on the substrate shown in Fig. 2, the substrate is not illustrated in Fig. 5. In other words, in Fig. 5, the sample S is illustrated including the substrate 401.

A tip portion of the sample retainer 412 is in contact with the sample S. As described earlier, the sample S contains a liquid and the sample retainer 412 is in contact with a surface of the liquid. The sample retainer 412 has an opening in the field of view 140. In other words, the sample retainer 412 is in contact with the sample S outside of the field of view 140.

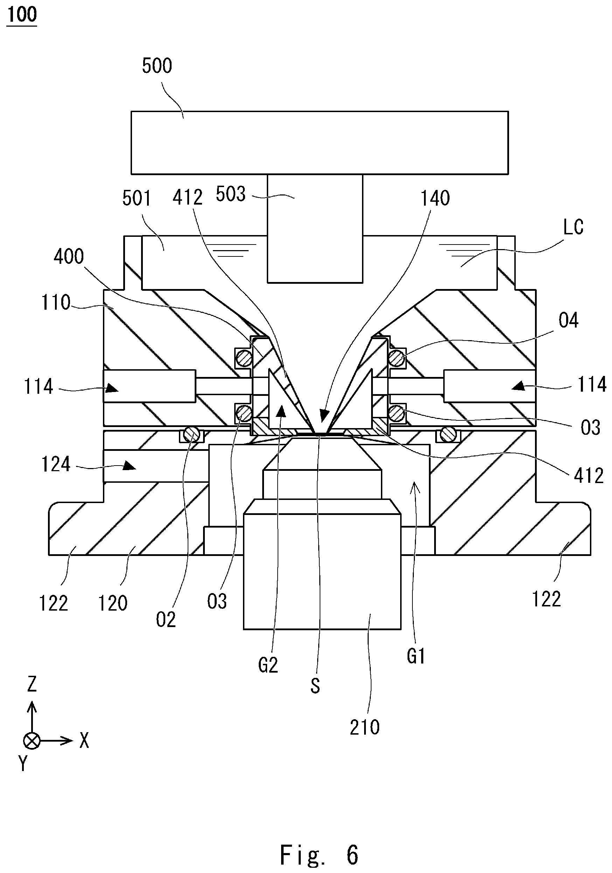

When the cryogen feeding apparatus 500 causes the cryogen 501 to drip into the cryogen storage tub 111, the cryogen 501 reaches the sample S. When the cryogen 501 comes into contact with the surface of the sample S, the sample S in the field of view 140 is frozen and fixed. Furthermore, as shown in Fig. 6, the cryogen feeding apparatus 500 may include a metal member 503 for maintaining a low temperature. The cryogen feeding apparatus 500 inserts the cooled metal member 503 into the cryogen storage tub 111. When the metal member 503 comes into contact with the cryogen 501, the cryogen 501 is maintained at a low temperature. The metal member 503 is a metal rod, a metal block, or the like cooled to the liquid nitrogen temperature. Liquid nitrogen may circulate inside the metal member 503. Bringing the metal member 503 into contact with the cryogen 501 enables the temperature of the cryogen 501 to be maintained at a low temperature. The metal member 503 constitutes a mechanism for maintaining low temperature after cryogen feed. In addition, the mechanism for maintaining the sample at a low temperature is not limited to the configuration shown in Fig. 6. For example, the sample holder 400 may be cooled by liquid nitrogen or the like.

The sample placement unit 100 on which the sample S is placed is installed on a stage of an inverted fluorescence microscope. The sample S contains cultured cells and a buffer solution. Pressure of a space around the sample S is adjusted to lower the height of the liquid level of the buffer solution to around 20 to 100 µm. Subsequently, cryofixation is performed by feeding a mixed cryogen of liquid propane and isopentane at an optional timing while performing wide field observation with the fluorescence microscope. After the cryofixation, the metal member 503 at the liquid nitrogen temperature is inserted into the cryogen storage tub and fluorescent observation is continued in a state where the low temperature is maintained. Since the sample S is fixed, exposure time can be extended and fluorescent observation can be performed with a high signal-to-noise ratio. Super-resolution fluorescence microscopic observation can also be performed over a long period of time.

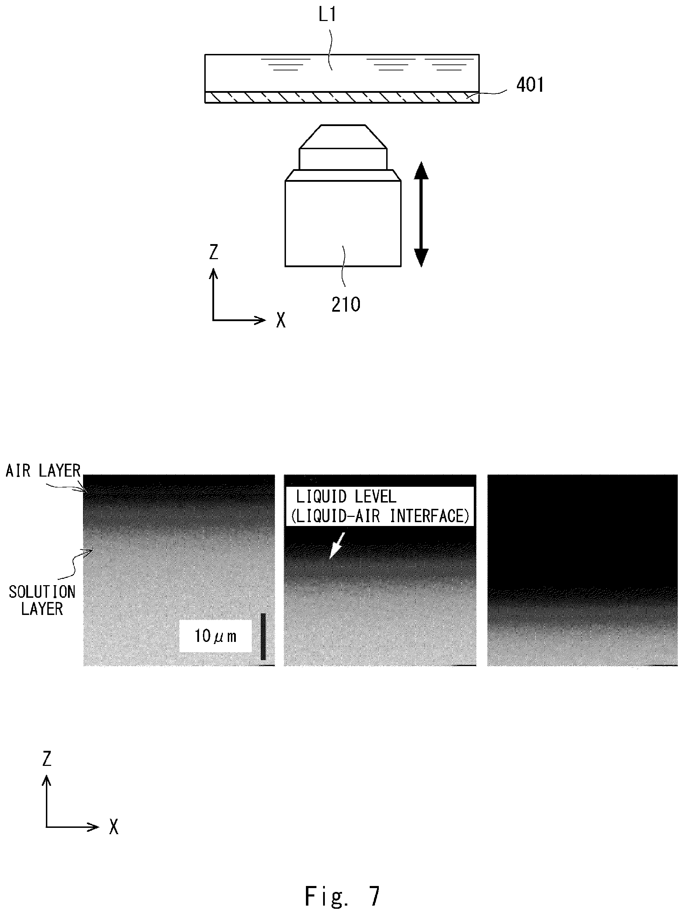

Fig. 7 is a diagram showing tomographic images captured at different heights of the liquid level. The tomographic images are measured by moving the objective lens 210 up and down. A fluorescent dye solution L1 is arranged on the substrate 401 that is a cover glass. The tomographic images are measured by a laser scanning fluorescence microscope.

In the tomographic images shown in Fig. 7, a lower layer represents a solution layer and an upper layer represents an air layer. Fig. 7 shows three tomographic images captured at different heights of the liquid level. Adjusting air pressure with the syringe 300 enables a height of an interface between the solution layer and the air layer (height of the liquid level) to be adjusted.

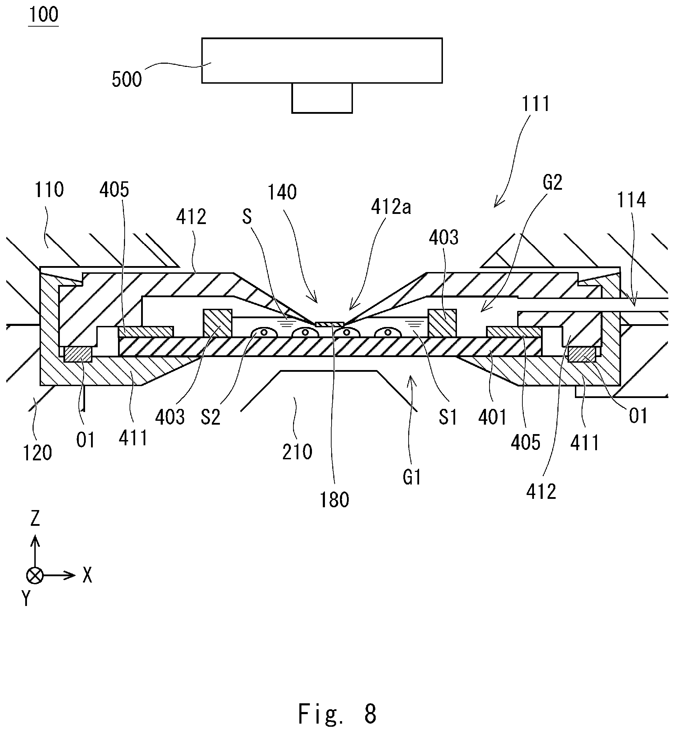

In addition, the height of the liquid level may be adjusted by means other than pressure adjustment. As shown in Fig. 8, the opening portion 412a of the sample retainer 412 may be provided with a pressing member 180. The pressing member 180 is held in a tip portion of the sample retainer 412. The pressing member 180 is formed of a material with high thermal conductivity such as copper or diamond. For example, a diamond substrate or a copper substrate can be used as the pressing member 180. The pressing member 180 may be a metal foil such as a copper foil. Using a thinner pressing member 180 enables freezing in a shorter period of time. Alternatively, the height of the liquid level may be adjusted using an absorbent material that absorbs liquids. For example, the absorbent material is brought into contact with a liquid inside the wall 403. Accordingly, since the liquid is absorbed by an absorbent material, the height of the liquid level can be lowered.

The height of the liquid level can be adjusted by pressing the pressing member 180 against the sample S. The height of the liquid level is lowered by pressing down more on the pressing member 180. **In** addition, the cryogen feeding apparatus 500 feeds the cryogen 501 once the height of the liquid level reaches a desired height. **In** other words, even if the cryogen feeding apparatus 500 supplies the cryogen 501 from above the pressing member 180, the cryogen 501 cools the sample S via the pressing member 180. The sample S is not limited to being placed on the substrate 401. For example, a micro flow path may be connected to a space between the pressing member 180 and the substrate 401 and a sample flowing in a visual field of observation may be observed. An area above the pressing member 180 constitutes a cryogen contact area with which the cryogen comes into contact.

Alternatively, the pressing member 180 may be a cooling block including an opening portion. The sample S containing an internal organ may be frozen by pouring the cryogen into the opening portion of the cooling block. **In** addition, low temperature may be maintained by the pressing member 180. Accordingly, the internal organ can be frozen and observed.

Using the pressing member 180 or the like enables the height of the liquid level to be adjusted without sucking gas inside the sample space G2. For example, a pressure balance is achieved between the sample space G2 and outside space in a state where the height of the liquid level is adjusted using the pressing member 180. The sample space G2 is sealed in a state where a pressure balance is achieved between the sample space G2 and outside space. Accordingly, since a pressure balance is maintained even when the pressing member 180 is removed, the height of the liquid level can be kept constant.

According to the present embodiment, cryofixation of a sample under microscopic observation at an optional timing can be performed with high reproducibility. Improvements in accuracy and reliability of sample analysis after freezing can also be expected. Since there is sufficient liquid outside the field of view 140, an environment in the periphery of the sample can be maintained. In addition, since the sample S can be accessed up to immediately before freezing, the sample S can be readily manipulated before freezing.

An observation method according to the present embodiment includes the steps of: arranging a sample including a liquid in a field of view of an objective lens; freezing, with a cryogen, the sample in the field of view of the objective lens in a state where a height of a liquid level of the sample in the field of view of the objective lens is made lower than outside of the field of view; and observing the frozen sample.

Furthermore, two or more types of cryogens may also be used. For example, it is possible to use a first cryogen that freezes the sample S or to use a second cryogen that maintains the frozen sample S in the frozen state. First, the first cryogen is supplied toward the sample S at room temperature. After the sample S is frozen, the first cryogen is stopped being supplied and the second cryogen is then supplied. The second cryogen maintains the sample S in a frozen state. Therefore, the sample S in the frozen state can be observed. The first cryogen and the second cryogen may be supplied from the same cryogen supply unit, or may be supplied from different cryogen supply units. Additionally, cooling means other than a cryogen may be used. For example, electronic cooling such as a Peltier element or the like can be used in combination.

As described above, supplying the cryogen toward the sample S allows the sample to freeze instantly. Accordingly, the sample can freeze while preserving the shape of the cells. Since the exposure time of a camera can be increased, the sample can be imaged with a high signal-to-noise ratio.

By simply introducing a cryogen at an optional timing, the sample S under microscopic observation can be fixed in a state close to its original state. Furthermore, the microscope according to the present embodiment is applicable to any type of optical microscopes or any optical response for observations, and therefore is applicable to all optical microscopes. The microscope according to the present embodiment allows for capturing a snapshot of the dynamics of a biological sample, for which it is difficult to integrate signals over a long period of time. The microscope 10 described above is applicable to a research in which biological samples are observed. Furthermore, the above-mentioned microscope 10 can also be applied clinically. A snapshot of molecular and ionic dynamics of a biological sample can be captured with high reproducibility and intactness.

In addition, how a sample is frozen can be observed. For example, the cryogen may be supplied while the user is observing the sample. As described earlier, the user observing the sample can adjust a freeze timing. Alternatively, the freeze timing can also be adjusted using various signals.

The present embodiment is applicable not only to inverted microscopes, upright microscopes, and stereomicroscopes, but also to other types of microscopes. For example, the present embodiment is also applicable to a microscope providing side illumination in which illumination light is incident to the sample from the side of the objective lens. The microscope is suitable for observing biological samples such as cultured cells and cardiomyocytes. Illumination light may be emitted from the side of the cryogen storage tub 111 after the sample is frozen.

The observation light to be observed with a microscope may be any responses of scattering, absorption, emission, and reflection. The microscope is also applicable to fluorescence observation and Raman scattering observation. The sample being observed under a microscope can be frozen with a cryogen, and the frozen sample can be continuously observed at low temperatures.

### Second Embodiment

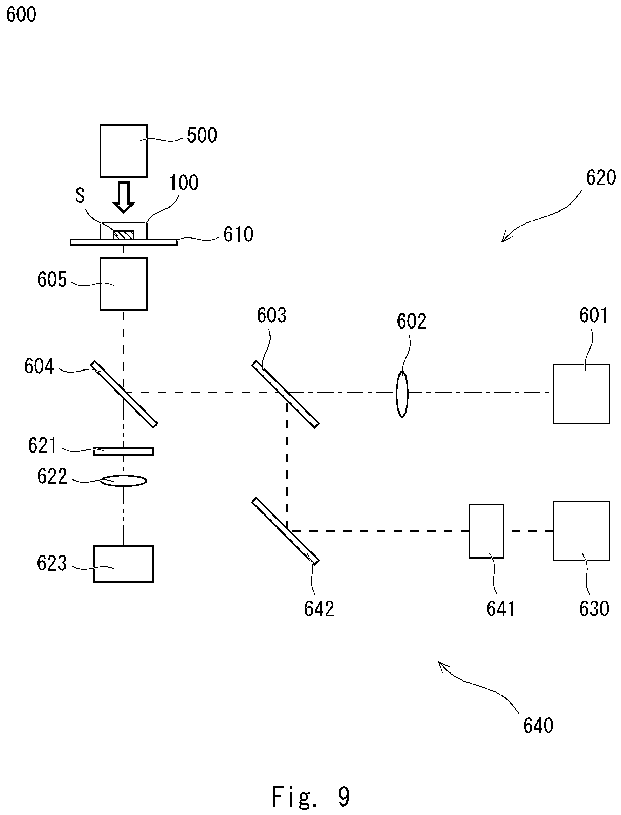

In a second embodiment, after the sample S is frozen with the cryogen 501, at least a part of the sample S is thawed by irradiating the sample S with laser light. After thawing, the thawed area is refrozen in an ambient cold environment and the refrozen portion is observed. By repeating thawing and refreezing, changes over time in the sample can also be observed. A microscope 600 according to the second embodiment will be described with reference to Fig. 9. Fig. 9 is a diagram showing a configuration of the microscope 600. Here, the microscope 600 will be described as a fluorescence microscope that detects fluorescence from the sample S.

The microscope 600 includes an observation light source 601, a stage 610, an observation optical system 620, a two-dimensional photodetector 623, a thawing light source 630, a thawing optical system 640, the sample placement unit 100, and the cryogen feeding apparatus 500. The observation optical system 620 includes a lens 602, a dichroic mirror 603, a dichroic mirror 604, an objective lens 605, a filter 621, and a tube lens 622. The thawing optical system 640 includes a shutter 641, a scanner 642, the dichroic mirror 603, the dichroic mirror 604, and the objective lens 605.

The stage 610 holds the sample placement unit 100 that accommodates the sample S. The sample placement unit 100 includes the sample holder 400 and the like shown in Fig. 1. Therefore, the sample S is placed on the stage 610. The cryogen feeding apparatus 500 is provided above the sample placement unit 100. The sample placement unit 100 includes adjustment means of a height of a liquid level in the visual field of observation described earlier. In addition, the cryogen feeding apparatus 500 includes means for dripping the cryogen 501 onto the sample S described earlier. After the height of the liquid level is adjusted by the sample placement unit 100, the cryogen is fed from the cryogen feeding apparatus 500 and the sample S freezes.

Next, the observation optical system 620 for guiding light from the observation light source 601 will be described. The observation light source 601 is, for example, a laser light source, an LED light source, or the like and generates excitation light for exciting the sample S. Laser light (excitation light) from the observation light source 601 is concentrated by the lens 602 and is incident to the dichroic mirror 603. The dichroic mirror 603 has wavelength characteristics of transmitting light with the excitation light wavelength and reflecting light for thawing.

The laser light transmitted through the dichroic mirror 603 is incident to the dichroic mirror 604. The dichroic mirror 604 has wavelength characteristics of reflecting light with the excitation light wavelength and transmitting fluorescence. The excitation light reflected by the dichroic mirror 604 is incident to the objective lens 605. The objective lens 605 irradiates a portion of the visual field of observation of the sample S with the excitation light.

The sample S is placed on a substrate (not illustrated) that transmits the wavelengths of excitation light, fluorescence, and thawing light. The sample S and the substrate are placed on the stage 610. The stage 610 includes an opening below the sample S portion. Therefore, the excitation light passes through the opening of the stage 610 and is incident to the sample S.

When the excitation light excites the sample S, fluorescence is generated from the sample S. Fluorescence from the sample S is refracted by the objective lens 605 and is incident to the dichroic mirror 604. The fluorescence is transmitted by the dichroic mirror 604 and is incident to the filter 621.

The filter 621 has wavelength characteristics that block excitation light from the observation light source 601 and light from the thawing light source 630. The filter 621 transmits the fluorescence generated by the sample S. The fluorescence transmitted through the filter 621 is incident to the tube lens 622. The tube lens 622 concentrates the fluorescence onto the two-dimensional photodetector 623. The two-dimensional photodetector 623 can capture a fluorescence image of the sample S.

The thawing light source 630 generates, for example, infrared light for heating. Although the thawing light source 630 will be described as a laser light source that generates laser light, the thawing light source 630 may also be a lamp light source or the like. Laser light from the thawing light source 630 is incident to the scanner 642 via the shutter 641. The scanner 642 is an optical scanner such as a galvanometer mirror. The scanner 642 is a two-axis scanner which two-dimensionally scans the irradiation position of the laser light on the sample S. Accordingly, any position of the sample S can be irradiated with the laser light.

The light reflected by the scanner 642 is incident to the dichroic mirror 603. The dichroic mirror 603 has wavelength characteristics that reflect infrared light. Therefore, the dichroic mirror 603 reflects the laser light from the scanner 642. Thereby, the laser light from the thawing light source 630 is propagated coaxially with the excitation light from the observation light source 601. The laser light reflected by the dichroic mirror 603 is incident to the dichroic mirror 604.

The dichroic mirror 604 has wavelength characteristics that reflect infrared light toward the objective lens 605. Therefore, the laser light reflected by the dichroic mirror 604 is incident to the objective lens 605. The objective lens 605 refracts the laser light so as to concentrate it onto the sample S. Irradiating the sample S with laser light partially heats the sample S, which has been frozen with the cryogen. Thereby, at least a portion of the sample S can be melted.

Additionally, the laser light from the thawing light source 630 is coaxial with the excitation light from the observation light source 601. Therefore, the laser light and the excitation light are incident to the sample S at the same position. Accordingly, a fluorescence image of the sample S at the position where the sample is thawed by the laser light can be captured. This then allows for observing the behavior of the frozen sample S while it thaws. Furthermore, the scanner 642 that scans the laser light is provided, allowing for controlling the irradiation position of the laser light. Accordingly, the sample S can be appropriately thawed. Additionally, it is also possible to control the position and time of thawing during observation.

Furthermore, the shutter 641 is arranged in the optical path of the laser light from the thawing light source. The shutter 641 is provided in an openable and closable manner. When the shutter 641 opens, the laser light is incident to the sample S to heat the sample S. When the shutter 641 closes, the sample S is no longer irradiated with the laser light. Controlling the opening and closing of the shutter 641 allows the sample S to be melted at an appropriate timing. By closing the shutter 641 and stopping laser light irradiation from the thawing light source after thawing, the thawed portion can be refrozen in an ambient cold environment and the sample S in a frozen state can be observed. In addition, by repeating thawing and refreezing, changes over time in the sample can also be observed.

Furthermore, intensity of laser light can be controlled in order to prevent the temperature of the sample S from rising after thawing. For example, the temperature of the sample S may be controlled by subjecting the laser light to intensity modulation. A temperature sensor may be provided on the sample or around the sample and feedback control of laser light intensity may be performed based on a detection result of the temperature sensor. Accordingly, the temperature of the sample S can be prevented from excessively rising after thawing.

### Third Embodiment

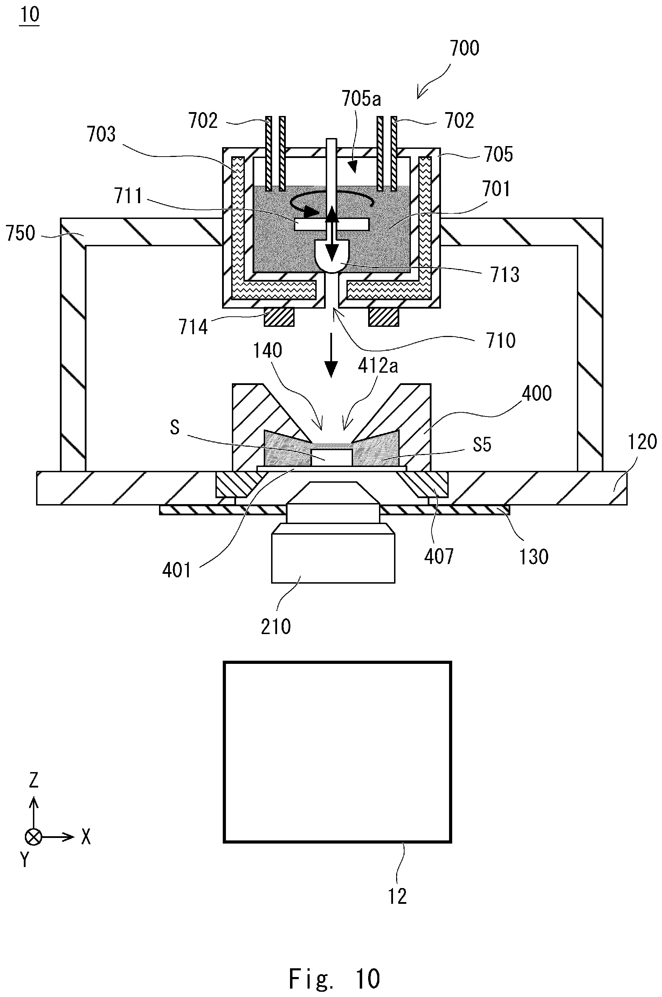

A cryogen manufacturing apparatus and a microscope according to a third embodiment will be described with reference to Fig. 10. Fig. 10 is a side cross-sectional view schematically showing an overall configuration of the microscope 10 including a cryogen feeding apparatus 700. In the present embodiment, the cryogen feeding apparatus 700 is installed above the sample S. The cryogen feeding apparatus 700 functions as a freezing apparatus for freezing the sample S. Since basic configurations of the microscope 10 are similar to those in the first and second embodiments, description thereof will be omitted when appropriate. For example, since configurations similar to those used in the first embodiment or the second embodiment can be adopted as the configurations of the microscope main body 12, the objective lens 210, and the like, detailed descriptions and illustrations will be omitted when appropriate.

The cryogen feeding apparatus 700 includes a cryogen container 705 and cooling means 703. The cryogen container 705 includes a storage tub 705a for storing a liquid cryogen 701. A space inside the cryogen container 705 constitutes the storage tub 705a for storing the cryogen 701. The cryogen container 705 may be a heat insulating container.

A release port 710 that releases the cryogen 701 toward the sample S is provided on a bottom surface of the cryogen container 705. The release port 710 is provided directly above the field of view 140. In addition, the cryogen 701 stored in the cryogen container 705 is released from the release port 710 toward the sample S in the field of view 140. Accordingly, cryofixation of the sample S can be performed during observation or immediately before observation.

The cryogen feeding apparatus 700 includes an open/close valve 713 for opening and closing the release port 710. The open/close valve 713 is provided so as to be movable in the z-direction in the storage tub 705a. The release port 710 is closed when the open/close valve 713 descends until the open/close valve 713 comes into contact with the bottom surface of the cryogen container 705. The release port 710 is opened when the open/close valve 713 ascends and separates from the bottom surface. When the open/close valve 713 opens, the cryogen 701 is supplied to the sample S from the release port 710. Accordingly, the sample S is frozen and fixed. The user may open or close the open/close valve 713 at any timing or open/close timings may be controlled by various signals.

An introduction path 702 is connected to the cryogen container 705 to supply material for the cryogen 701. The introduction path 702 is a piping that is attached to the cryogen container 705. The material of the cryogen 701 passes through the introduction path 702 and is introduced to the storage tub 705a of the cryogen container 705. Examples of the material of the cryogen 701 include a liquid such as isopentane and a gas such as propane. In this case, the cryogen container 705 is provided with two introduction paths 702. Isopentane is supplied from one of the introduction paths 702 and propane is supplied from the other introduction path 702. Obviously, the material of the cryogen is not limited thereto. In addition, there may be one material of the cryogen instead of two materials.

The cryogen container 705 is provided with the cooling means 703 that cools the material of the cryogen 701. For example, the cryogen 701 is generated in the cryogen container 705 as a result of the cooling means 703 cooling the material introduced from the introduction path 702. For example, the cryogen 701 is generated when propane is cooled and liquefies in the storage tub 705a. In this case, the cooling means 703 is liquid nitrogen. For example, the cryogen container 705 is a double container with liquid nitrogen filled between inner and outer containers and liquid nitrogen is added as needed.

The cooling means 703 is arranged so as to surround the periphery of the storage tub 705a. The cooling means 703 cools the material of the cryogen and generates the cryogen 701. Furthermore, the cooling means 703 maintains the cryogen 701 in the cryogen container 705 at a low temperature. Note that the cooling means 703 is not limited to liquid nitrogen and may be a cooling machine including a compressor or the like. In other words, the cooling means 703 may include a cooling machine that cools the cryogen or the container. Furthermore, the cooling means 703 may include both liquid nitrogen and a cooling machine.

In addition, the cryogen container 705 is provided with a stirring mechanism 711. The stirring mechanism 711 stirs the material of the cryogen 701 introduced into the storage tub. The stirring mechanism 711 stirs the material of the cryogen 701 by rotating a stirring blade around a shaft. A cryogen can be generated in an efficient manner by having the stirring mechanism 711 stir the material of the cryogen 701.

In this manner, the cryogen feeding apparatus 700 functions as a cryogen manufacturing apparatus that manufactures the cryogen 701 while positioned directly above the sample S. The cryogen 701 manufactured in the cryogen container 705 is released from the release port 710 toward the sample S present in the field of view 140. Accordingly, the sample S can be frozen and observed. Obviously, the material of the cryogen 701 may be other substances. In addition, the cryogen 701 itself may be directly introduced to the cryogen container 705 from the introduction path 702. In other words, the introduction path 702 is provided for introducing the cryogen or a material thereof into the cryogen container 705.

The cryogen feeding apparatus 700 is held by a holder 750. The holder 750 is an enclosure that supports the cryogen container 705. In other words, the holder 750 holds the cryogen feeding apparatus 700 so that the cryogen feeding apparatus 700 is arranged above the sample S. While the holder 750 is attached to the second plate 120 in Fig. 10, the holder 750 may be attached to something other than the second plate 120. For example, the holder 750 may be provided separately from the microscope main body 12 or the stage portion thereof. For example, the holder 750 may be fixed to a wall surface or a column in a room that is a use environment of the microscope 10. The cryogen feeding apparatus 700 may be provided separately from the microscope 10. Accordingly, when the cooling means 703 is a cooling machine including a compressor or the like, vibration of the cooling means 703 can be prevented from being transmitted to the sample S. As a result, observations can be performed in a stable manner.

In addition, the cryogen container 705 may be provided with an illumination light source 714. The illumination light source 714 is an LED light source or the like and generates illumination light for illuminating the sample S. For example, the illumination light source 714 is a ring illumination mounted to the bottom surface of the cryogen container 705 or the like. The illumination light source 714 is arranged around the release port 710. Therefore, the cryogen 701 released from the release port 710 passes through a hollow portion of the illumination light source 714 and reaches the sample S.

In Fig. 10, the sample holder 400 is placed on the second plate 120. Note that the sample holder 400 may be placed between the first plate 110 and the second plate 120 in a similar manner to the first and second embodiments.

The sample S is arranged on the substrate 401. The substrate 401 is fixed on the second plate 120. The sample holder 400 holds the sample S on the substrate 401. The sample holder 400 is provided with the opening portion 412a that corresponds to the field of view 140. The cryogen 501 released from the release port 710 passes through the opening portion 412a and comes into contact with the sample S. Accordingly, the sample S in the field of view 140 is frozen and fixed.

A part of the sample S in the sample holder 400 is immersed in a fluid S5. The fluid S5 is fed into a holding space surrounded by the sample holder 400. The sample holder 400 functions as a wall for stemming the fluid S5. As shown in the first embodiment, the fluid S5 is a buffer solution or a culture medium.

Furthermore, the second plate 120 is provided with a temperature adjustment mechanism 407. The temperature adjustment mechanism 407 adjusts the temperatures of the sample S and the fluid S5. For example, the temperature adjustment mechanism 407 adjusts the temperatures of the sample S and the fluid S5 to a temperature suitable for acetone replacement. For example, the temperature adjustment mechanism 407 includes a heater, a cooling mechanism for maintaining a low temperature, and the like. First, the temperature adjustment mechanism 407 maintains the sample S and the fluid S5 at a low temperature of around -90°C. In addition, the temperature adjustment mechanism 407 raises the temperatures of the sample S and the fluid S5 to room temperature over a period of about one day. While the temperature adjustment mechanism 407 is in contact with the bottom surface of the sample holder 400, the position of the temperature adjustment mechanism 407 is not particularly limited. In addition, the temperature adjustment mechanism 407 may be used to thaw the frozen sample S.

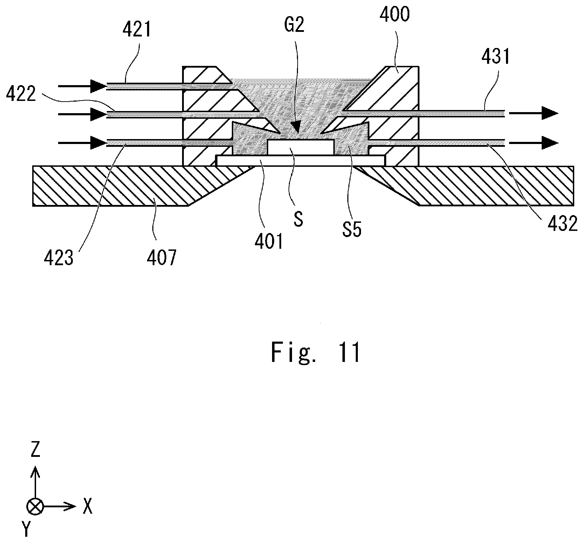

The sample holder 400 may be provided with a mechanism for supplying and discharging the fluid S5. A configuration of the sample holder 400 capable of supplying or discharging the fluid S5 will be described with reference to Fig. 11. Fig. 11 is an XZ cross-sectional view showing a configuration of the sample holder 400 in an enlarged manner.

The sample holder 400 is provided with supply pipes 421 to 423 and discharge pipes 431 and 432. The supply pipes 421 to 423 penetrate a side wall of the sample holder 400. Therefore, the supply pipes 421 to 423 can supply a fluid to the sample space G2 in the sample holder 400. The discharge pipes 431 and 432 penetrate a side wall of the sample holder 400. Therefore, the supply pipes 421 to 423 can discharge the fluid S5 in the sample space G2 to outside.

For example, acetone is supplied to the sample holder 400 from the supply pipe 421. By supplying acetone to the sample space G2 in the sample holder 400, acetone replacement of the sample S is performed. Acetone replacement dehydrates and chemically fixes the sample S. Water is supplied to the sample holder 400 from the supply pipe 423. For example, a volume of water in the sample space G2 can be controlled by controlling pressure applied to the supply pipe 423. Therefore, the height of the liquid level of the sample S can be set to a desired height.

A staining dye or a decolorizing agent is supplied to the sample holder 400 from the supply pipe 422. By supplying the staining dye to the sample space G2 in the sample holder 400, the sample S is stained. By supplying a decolorizing agent to the sample holder 400, the stained sample S is decolorized. In addition, a plurality of lines of piping may be provided on an upstream side of the supply pipe 422 so that the decolorizing agent or the staining dye supplied to the sample holder 400 can be switched between each other. Switching between fluorescent dyes to be supplied to the sample holder 400 enables multistaining to be performed using different fluorescent types. In this manner, the supply pipe 422 can supply a chemical solution such as a staining dye to the sample S.

The discharge pipes 431 and 432 discharge a fluid in the sample space G2 to outside of the sample holder 400. The discharge pipes 431 and 432 are connected to a syringe or the like. In addition, by sucking gas with the syringe or the like, the fluid S5 in the sample space G2 can be discharged to the outside of the sample holder 400.

An observation method according to the present embodiment will be described. First, the cryogen feeding apparatus 700 fabricates the cryogen 701 above the sample S. In other words, the material of the cryogen 701 is supplied to the cryogen container 705 from the introduction path 702. In addition, the cryogen 701 is fabricated in the cryogen container 705 as a result of the cooling means 703 cooling the material of the cryogen 701.

After the cryogen 701 is stored in the cryogen container 705, the open/close valve 713 opens to supply the cryogen 701 from the release port 710. The timing at which the open/close valve 713 opens may be selected by the user or determined by a signal from the sample S or the like. The sample S is frozen when the cryogen 701 comes into contact with the sample S or the fluid S5. In addition, the user observes the frozen sample S. Accordingly, the user can observe the frozen and fixed sample S.

After the observation ends, the sample S is thawed by heating provided by the temperature adjustment mechanism 407 or by irradiating light for thawing. Alternatively, after observing the sample S, the sample S may be replaced and fixed with acetone or the like. In addition, the sample S chemically fixed by acetone or the like is observed. Accordingly, both chemical fixation and cryofixation can be used with respect to the same sample S. Alternatively, after thawing the sample S by light irradiation or the like, the sample may be frozen and fixed to be observed once again. In this manner, fixation and observation of the sample S may be repeatedly performed.

In addition, the sample S may also be multistained using a plurality of fluorescent dyes. Hereinafter, an observation method when performing multistaining will be described. First, the cryogen feeding apparatus 700 fabricates the cryogen 701 above the sample S. Then, the cryogen 701 is supplied from the release port 710 to freeze the sample S. A chemical fixative such as acetone is supplied from the supply pipe 421 to the sample holder 400 to perform chemical fixation. For example, due to acetone replacement, the sample S is dehydrated and chemically fixed.

The sample S is stained by supplying a staining dye from the supply pipe 422 to the sample holder 400. For example, staining using an antibody or hybridization can be performed. RNA (Ribonucleic acid), a protein, or the like in the sample S may be stained by the staining dye. In addition, the user observes the stained sample S.

The stained sample S is decolorized by supplying a decolorizing agent from the supply pipe 422 to the sample holder 400. The decolorizing agent is supplied to the sample space G2 to decolorize the sample S. At this point, the staining dye, the decolorizing agent, or the like may be discharged from the discharge pipes 431 and 432.

Next, the sample S is stained by supplying a different staining dye from the supply pipe 422 to inside the sample space G2. In addition, the user observes the sample S stained by the different staining dye. In this manner, staining, observation, and decolorization of the sample S are repeatedly performed. Accordingly, sample images of different types of molecules individually stained using various staining dyes can be obtained. Using a plurality of staining dyes enables various substances in the sample S to be targeted for observation. For example, color fluorescent images in accordance with fluorescence wavelengths can be generated. In addition, the sample S can be observed after being subjected to cryofixation or chemical fixation. The processing described above can be performed in the field of view 140. Therefore, observations can be performed in a simplified manner.

### First Modified Example

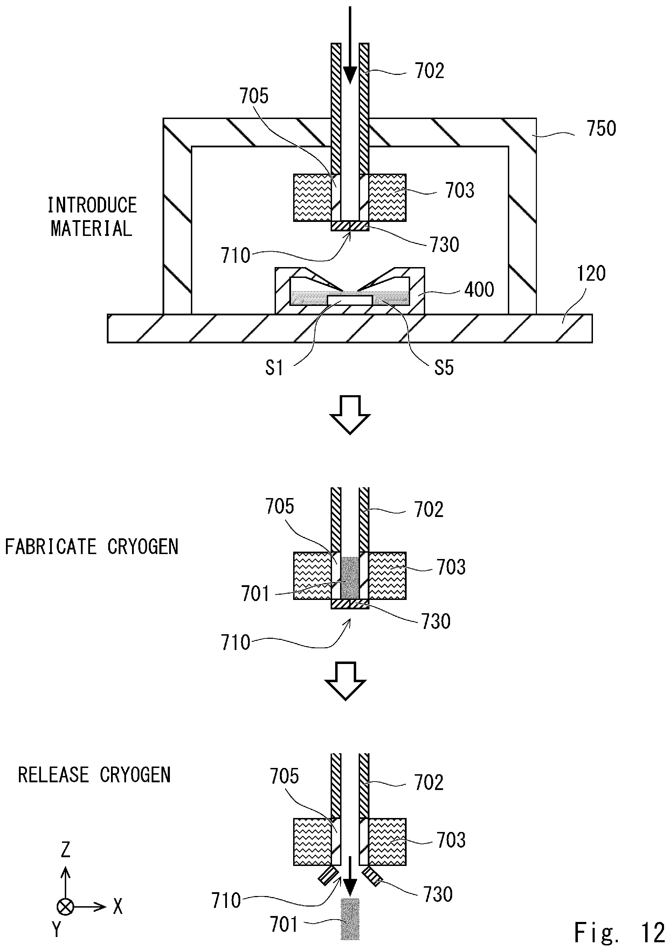

A configuration of the cryogen feeding apparatus 700 according to a first modified example will now be described with reference to Fig. 12. Fig. 12 is a diagram for describing a configuration and operations of the cryogen feeding apparatus 700. Since configurations other than that of the cryogen feeding apparatus 700 are similar to those in the embodiments described above, descriptions thereof will be omitted when appropriate. For example, as the configurations of the sample holder 400 and the microscope main body 12, the configurations shown in the first to third embodiments can be adopted.

Fig. 12 shows a step of introducing a material of a cryogen, a step of fabricating the cryogen, and a step of releasing the cryogen, respectively. Note that the sample holder 400 and the like are omitted in the step of fabricating the cryogen and the step of releasing the cryogen.

As shown in Fig. 12, the release port 710 provided in the cryogen container 705 is provided with a lid 730. The lid 730 is provided so as to cover the release port 710 of the cryogen container 705. The release port 710 is closed by the lid 730 prior to the release of the cryogen. The lid 730 is opened and closed due to pressure in the cryogen container 705. For example, raising the pressure in the cryogen container 705 opens the lid 730.

The material of the cryogen is fed into the cryogen container 705 from the introduction path 702. The cooling means 703 is provided around the cryogen container 705. Therefore, the material of the cryogen is cooled in the cryogen container 705 and the cryogen 701 is fabricated. In this case, a solid cryogen such as solid nitrogen is fabricated as the cryogen 701. Therefore, liquid nitrogen can be used as the material of the cryogen 701. The solid cryogen may be a cooled metal sphere, ice, solid carbon dioxide, or the like.

Once the cryogen 701 is fabricated in the cryogen container 705, a gas is introduced into the cryogen container 705 from the introduction path 702. Accordingly, the cryogen container 705 is pressurized and the cryogen 701 is pushed out from the release port 710. In other words, the lid 730 opens and the cryogen 701 falls from the release port 710. Even with such a configuration, the sample S can be frozen and fixed by a cryogen. Therefore, similar effects to those described above can be obtained. A gas outlet or a liquid outlet may also be added in addition to the introduction path 702 shown in Fig. 12.

### Fourth Embodiment

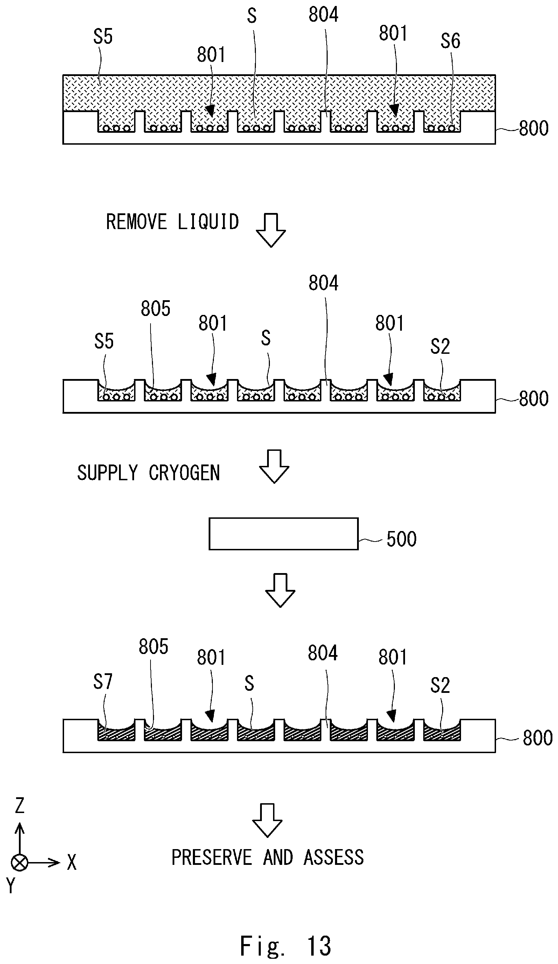

A sample freezing method according to a fourth embodiment will be described with reference to Fig. 13. Fig. 13 is a side cross-sectional view of a sample substrate 800 on which the sample S is placed and a diagram for describing procedures of the sample freezing method for freezing the sample S. The sample S contains a liquid such as a culture fluid and is placed on the sample substrate 800. For example, cells S6 are immersed in the fluid S5. For example, the fluid S5 is a culture fluid or the like and the cells S6 are floating cells, a cell mass, or the like.

A plurality of grooves 801 for holding the liquid sample S are formed on the sample substrate 800. Specifically, the sample substrate 800 includes a plurality of projecting portions 804 and a plurality of recessed portions 805. The projecting portions 804 and the recessed portions 805 are repetitively formed in the X direction. In addition, the recessed portions 805 constitute the grooves 801 for holding the liquid sample S. The sample substrate 800 is formed of a transparent material such as quartz.

The grooves 801 extend in the Y direction that is perpendicular to the paper surface. In addition, the plurality of grooves 801 are arranged in a row in the X direction. For example, a depth (size in the Z direction) of the grooves 801 ranges from 50 µm to 100 µm. In addition, a width (size in the Y direction) of the grooves 801 ranges from 100 µm to 300 µm. Note that the depth and the width of the grooves 801 (recessed portions) are not limited to the values described above and need only be sizes capable of holding cells or the like. In addition, while the depths and widths of all of the grooves 801 are the same in Fig. 13, the grooves 801 may be provided in different sizes.

A planar shape of irregularities may be a slit array, a hole array, or a knitted shape. For example, the sample substrate 800 may be provided with a slit array including a plurality of grooves arranged in a single row. The sample substrate 800 may be provided with a hole array including a plurality of holes two-dimensionally arranged in XY directions. Furthermore, mesh-shaped recessed portions including a plurality of grooves formed in the two directions of the X direction and the Y direction, respectively, may be provided. In addition, the plurality of recessed portions 805 may be partially connected to each other. In this case, the height of the fluid S5 in the plurality of recessed portions 805 can be aligned.

In addition, bottom surfaces of the recessed portions 805 may have holes to allow the fluid S5 to pass through. Providing holes on the bottom surfaces enables a volume of the fluid S5 to be reduced. Furthermore, the sample substrate 800 is not limited to quartz and may be a material with flexibility such as resin.

A method of freezing the sample S will be described. First, the sample substrate 800 including the plurality of recessed portions 805 is prepared. The liquid sample S is arranged in the plurality of recessed portions 805 of the sample substrate 800. The fluid S5 contains a plurality of cells.

The fluid S5 on top of the sample substrate 800 is removed. For example, a part of the fluid S5 in the grooves 801 is removed by tilting the sample substrate 800. Accordingly, a volume of the fluid S5 in the grooves 801 can be reduced. The liquid volume of the fluid S5 can be reduced by vaporizing or evaporating the fluid S5. Alternatively, the liquid volume of the fluid S5 can be reduced by sucking the fluid S5. At this point, the cells S6 are held in the grooves 801.

Then, a cryogen is supplied to the sample S to freeze the sample S. For example, the cryogen feeding apparatus 500 is provided above the sample substrate 800. The cryogen feeding apparatus 500 releases the cryogen toward the sample substrate 800. Accordingly, a frozen sample S7 in which the cells S6 and the fluid S5 are frozen is formed in the grooves 801. In this case, the cryogen is supplied from above in a similar manner to the first to third embodiments. The cryogen may be a liquid or a solid. The cells S6 can be frozen by bringing the cryogen into contact with the sample S. Then, the frozen sample S7 is preserved and assessed.

As described above, the sample substrate 800 with irregularities (bumps and dips) on its surface is prepared. Cells or a cell group is placed in the recessed portions. Removing the fluid S5 on the sample substrate 800 before freezing enables a thickness of the solution in a vicinity of the cells to be reduced to several 10 µm to several 100 µm. After removing the liquid on the sample substrate 800, a liquid or solid cryogen is brought into contact with the cells from above. Accordingly, the cells or the like can be frozen quickly. The frozen sample S7 may be observed as in the first to third embodiments.

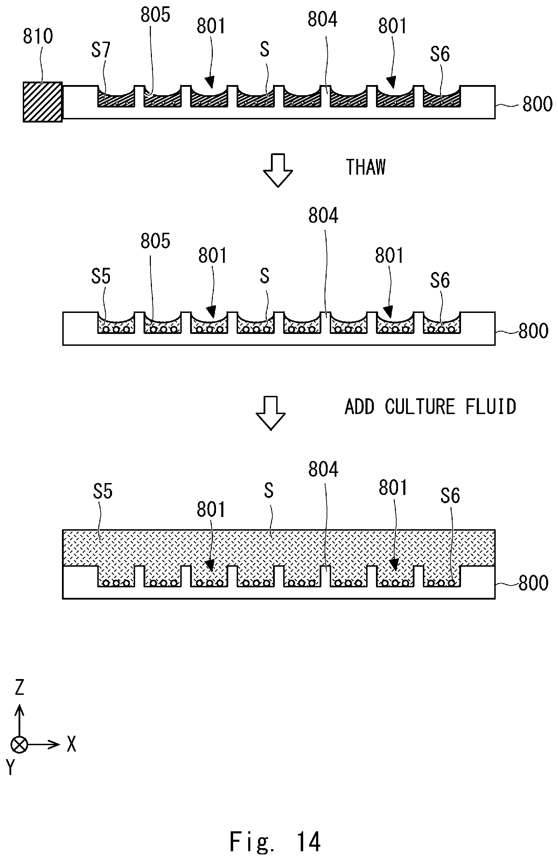

Furthermore, as shown in Fig. 14, the frozen sample S can also be thawed. Fig. 14 is a side cross-sectional view showing procedures for thawing the frozen sample S7. For example, the frozen sample S7 is thawed by bringing a heat source 810 into contact with a rear surface or a side surface of the sample substrate 800. A solution or a solid can be used as the heat source 810. The sample substrate 800 or the frozen sample S7 is heated with the heat source 810 to thaw the frozen sample S7. The sample substrate 800 may be entirely or partially constituted of or coated by a metal. Bringing the heat source 810 into contact with the metal portion enables thawing to be performed at a higher speed.

In addition, light, microwaves, or the like can be used as the heat source. The frozen sample S7 may be thawed by irradiating the frozen sample S7 with laser light, microwaves, or the like. A method of thawing the frozen sample S7 is not particularly limited. Furthermore, as shown in Fig. 14, the cells S6 are cultured by adding a culture fluid or the like to the thawed sample S. Accordingly, the cells S6 contained in the sample S once frozen can be cultured again.

Damage to the cells during freezing and thawing of the cells can be suppressed. The liquid volume around the cells can be reduced while the cells remain on the sample substrate 800. In addition, a cryogen is brought into contact with the cells to freeze the cells in a state where the liquid volume has been reduced. Keeping the liquid volume low enables a rate of cell freezing to be increased and the probability of ice crystal formation to be significantly reduced. Since the liquid volume is smaller than normal cryopreserved cells, rapid thawing can be performed. Therefore, ice crystal formation during thawing can be prevented.

Even in the thawing process, bringing the heat source 810 into contact with the cells or generating heat by light irradiation enables a change from a cryopreservation temperature to room temperature to be made in a short period of time. Even in the thawing process, ice crystal formation can be suppressed and the probability of cell damage can be reduced.

Providing a surface of the sample substrate 800 with microstructures enables the solution around the cells to be reduced without moving the cells. While cells are to flow together with the solution on a flat substrate, cell outflow can be prevented with a sample substrate with fine irregular structures.

Freezing can be performed immediately from a cell culture environment. In addition, a culture can be started immediately after thawing. Furthermore, the frozen cells have a plate shape and a volume of the frozen cells is smaller than conventional preservation in a solution. Therefore, a larger number of cells can be preserved in a smaller storage volume and temperature control is facilitated.

In addition, the sample substrate 800 may be placed on a stage of the microscope 10. A cryogen is supplied with respect to the sample substrate 800 placed on the stage. Accordingly, since the cells S6 can be frozen in the field of view of the microscope 10, a situation of the sample S can be observed during freezing. In other words, the user can observe how the cells S6 freeze.

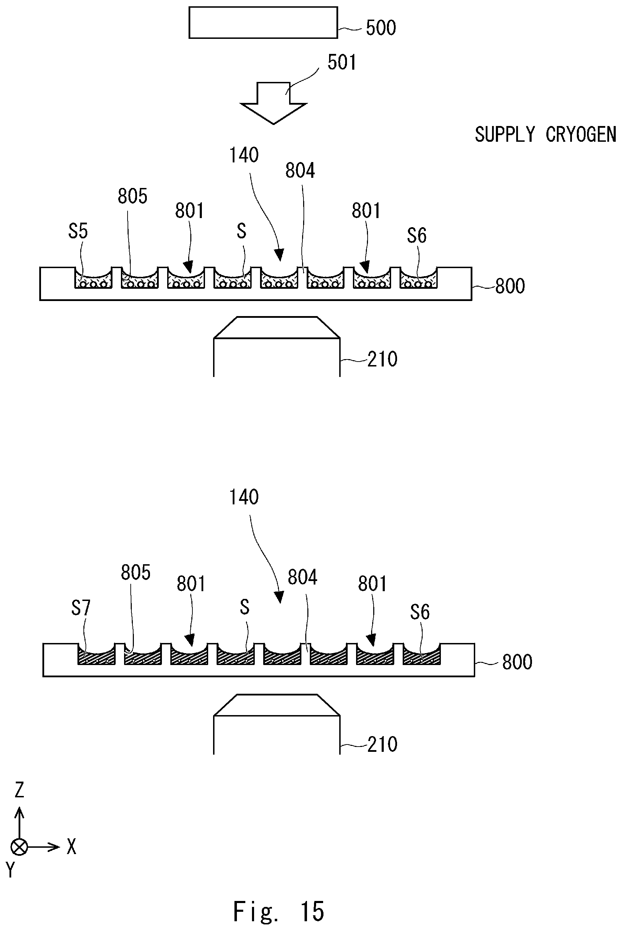

Fig. 15 is a side cross-sectional view showing a situation where the sample S during freezing is observed. The sample substrate 800 is arranged in the field of view 140 of the objective lens 210. At this point, the objective lens 210 is installed directly under the sample substrate 800. The cryogen 501 is supplied from above with respect to the sample S in the field of view 140 during observation by the user. Accordingly, the sample S is frozen and the frozen sample S7 is formed. The user can observe a situation of the sample S during freezing.

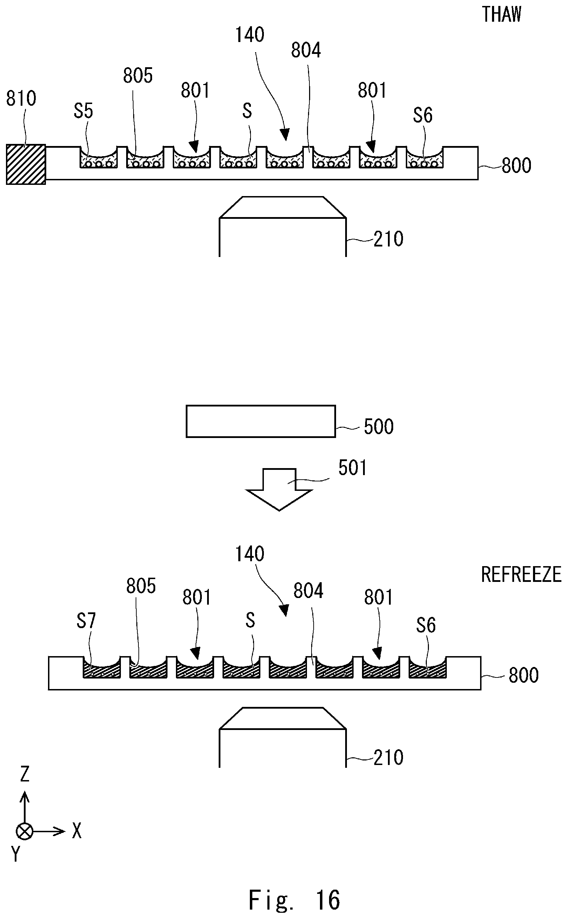

Furthermore, as shown in Fig. 16, bringing the heat source 810 in contact with the sample S on the microscope enables microscopic observation of a situation during freezing to be performed. In addition, refreezing can be performed by removing the heat source 810. Accordingly, the sample S can be repeatedly frozen and thawed and the situation of the sample S during repeated freezing and thawing can be observed under a microscope. Cells during freezing, storage, and thawing can be readily microscopically observed. Cryo-optical observation, assessment of frozen cells, and cell observation during/after thawing can be performed.

When observing with a microscope, the sample substrate 800 is preferably a quartz substrate. Accordingly, since distortion of the sample substrate 800 due to temperature changes can be reduced, blurring or misalignment of an observed image can be prevented during freezing while performing microscopic observation.

The fluid S5 preferably contains metallic colloids such as gold colloids or metallic particles. In addition, a cryogen may contain metallic colloids such as gold colloids or metallic particles. Accordingly, efficiency of cooling and efficiency of thawing can be further increased. A frozen sample can be kept and preserved at a low temperature by bringing each sample substrate into contact with a cryogen or a cooled solid. In addition, the fluid S5 and the cryogen may include microparticles such as dielectric nanoparticles which decrease in volume at low temperatures. Accordingly, even if ice crystals form in the fluid S5, the volume increase of the fluid S5 due to ice crystal formation can be reduced and deformation of the sample due to freezing can be suppressed.

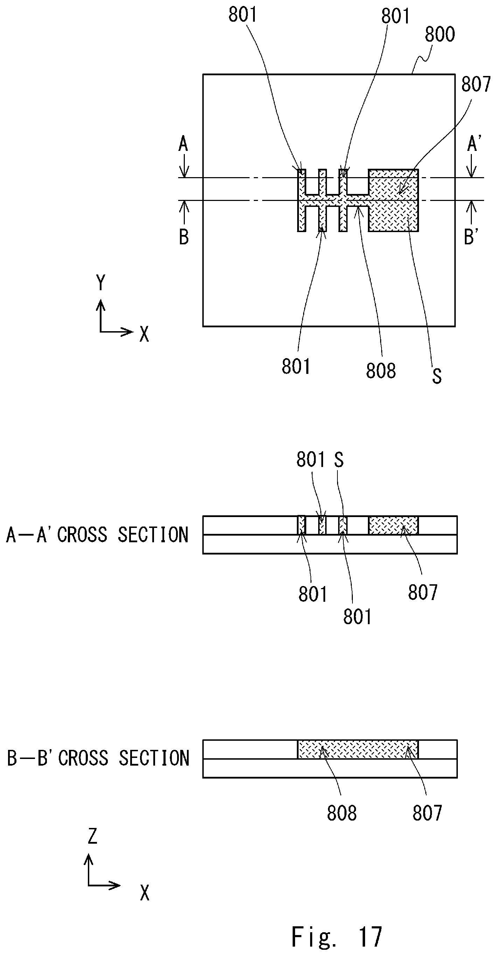

A solution reservoir or a cover to prevent liquid reduction due to water evaporation may be provided. Fig. 17 is a plan view and a cross-sectional view showing the sample substrate 800 provided with a reservoir. The sample substrate 800 includes a plurality of grooves 801. Each groove 801 extends in the Y direction. In addition, the plurality of grooves 801 are connected to a reservoir 807. Specifically, a groove 808 extends in the X direction from the reservoir 807. The groove 808 is a recessed portion that extends in the X direction. Three grooves 801 are connected by the groove 808. In other words, the groove 808 intersect with the grooves 801.

The reservoir 807 has a sufficiently wider area than the grooves 801 and 808. Therefore, a reduction in a liquid volume of the grooves 801 due to evaporation of liquid can be suppressed. Accordingly, freezing, preservation, or observations can be performed in a stable manner. Furthermore, the sample substrate 800 may be provided with a cover in order to prevent a reduction in the liquid volume due to evaporation. For example, the cover is provided so as to cover the reservoir 807, the grooves 801, or the groove 808. Obviously, the cover may only cover a part of the reservoir 807 or the like.

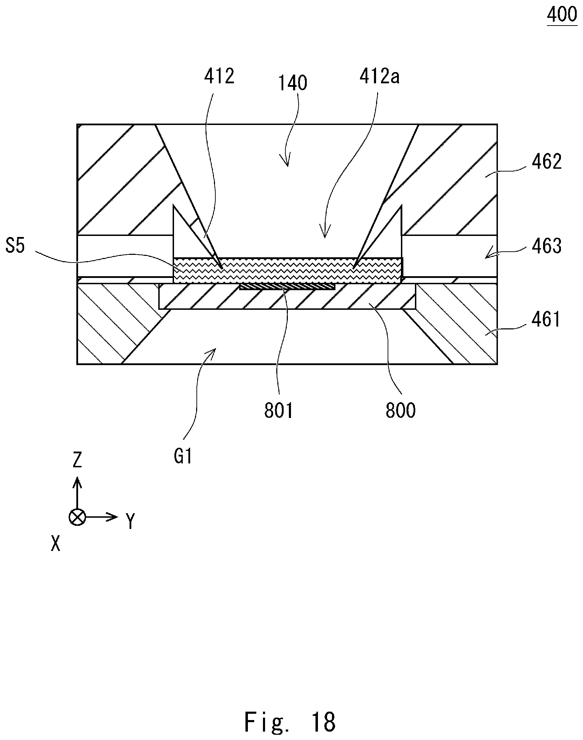

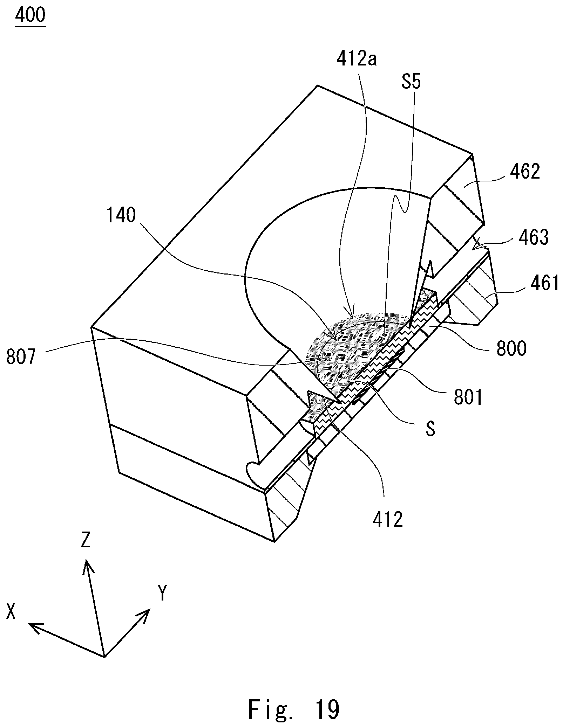

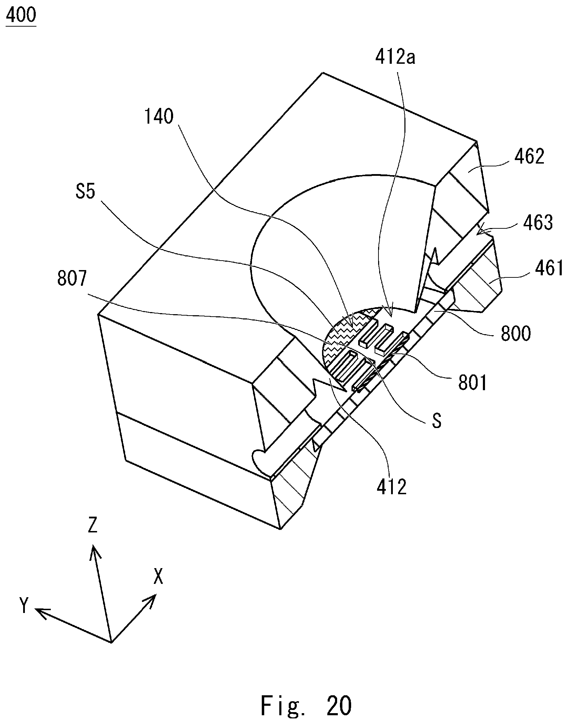

A configuration of the sample holder 400 that holds the sample substrate 800 will be described with reference to Figs. 18 to 20. Fig. 18 is a YZ cross-sectional view showing a configuration of the sample holder 400. Figs. 19 and 20 are cross-sectional perspective views of the sample holder 400. Fig. 19 shows a configuration before adjustment of a liquid volume and Fig. 20 shows a configuration after adjustment of the liquid volume.

The sample holder 400 includes an upper holder 462 and a lower holder 461. The upper holder 462 is arranged above the sample substrate 800. The lower holder 461 is arranged below the sample substrate 800. The sample substrate 800 is arranged between the upper holder 462 and the lower holder 461. The upper holder 462 and the lower holder 461 are plate-shaped members, respectively, and are arranged so as to oppose each other.

The objective lens space G1 for arranging the objective lens (not illustrated) is formed in the lower holder 461. The sample retainer 412 is formed in the upper holder 462. The sample retainer 412 is in contact with the liquid S1 outside of the field of view 140. The sample retainer 412 is provided with the opening portion 412a. A cryogen is supplied from above the upper holder 462. Therefore, the cryogen passes through the opening portion 412a and comes into contact with the sample S.

In addition, a suction port 463 is formed to the side of the upper holder 462. The suction port 463 is a through-hole that penetrates the upper holder 462 and extends in the Y direction. The suction port 463 is connected to a syringe or the like provided outside of the sample holder 400. A height of a liquid level can be adjusted by sucking the fluid S5 from the suction port 463. In other words, a liquid volume can be reduced by sucking the fluid S5 from the suction port 463. Reducing the liquid volume enables the sample S to be frozen in a short period of time after supplying the cryogen.

While the surface of the sample substrate 800 is exposed when the fluid S5 is sucked, the fluid S5 remains in the grooves 801. Adjusting the depth of the grooves 801 in a similar manner to the fourth embodiment enables a thickness of a remaining solution to range from several 10 µm to several 100 µm. Accordingly, supplying the cryogen enables the sample S to be frozen rapidly and with high reproducibility. In addition, the sample S is cells or the like and is held in the grooves 801 in a similar manner to the fourth embodiment. Furthermore, the width of the grooves 801 may be made sufficiently large so that the thickness of the residual solution ranges from several 10 µm to several 100 µm only in the observation area in a center portion of the grooves 801 and the thickness of the residual solution near a sidewall surface of the grooves 801 is thicker than in the observation area in the center portion. Accordingly, a reduction in the liquid volume in the observation area due to evaporation can be suppressed more efficiently.

As shown in Figs. 18 and 19, first, the sample S containing a sufficient amount of the fluid S5 is placed on the sample holder 400. At this point, an amount of the fluid S5 that overflows from the grooves 801 may be dripped onto the sample substrate 800. In addition, the fluid S5 is sucked from the suction port 463. Accordingly, since the liquid volume decreases as shown in Fig. 20, the liquid level drops. A surface of the sample substrate 800 is exposed in a portion excluding the grooves 801 and the like. After sucking the fluid S5 from the suction port 463, the cryogen is supplied through the opening portion 412a. Accordingly, since the cryogen comes into contact with the sample S, the sample S can be frozen. Since the liquid volume has been reduced as described above, the sample S can be frozen quickly. The frozen sample is observed in the field of view 140.

In this case, the fluid S5 is discharged from the suction port 463 provided in the Y direction. Therefore, the liquid level is the lowest on an end portion side in the Y direction. The fluid S5 on the sample substrate 800 is completely sucked in a vicinity of the suction port 463. On the other hand, since the fluid S5 on the sample substrate 800 is not completely sucked on an end portion side in the X direction, the liquid level rises.

In this manner, depending on an arrangement of the suction port 463, a distribution arises in the height of the liquid level. The sample S can be prevented from drying by making heights of the liquid level uneven. Even when drying progresses with time, the fluid S5 remains at both ends in the X direction. Furthermore, as shown in Fig. 20, drying can be prevented in an effective manner by providing the reservoir 807 on the end portion side in the Y direction. In other words, providing the reservoir 807 on a side separated from the suction port 463 enables a sufficient amount of the fluid S5 to be stored in the reservoir 807.

### (Sample holder)

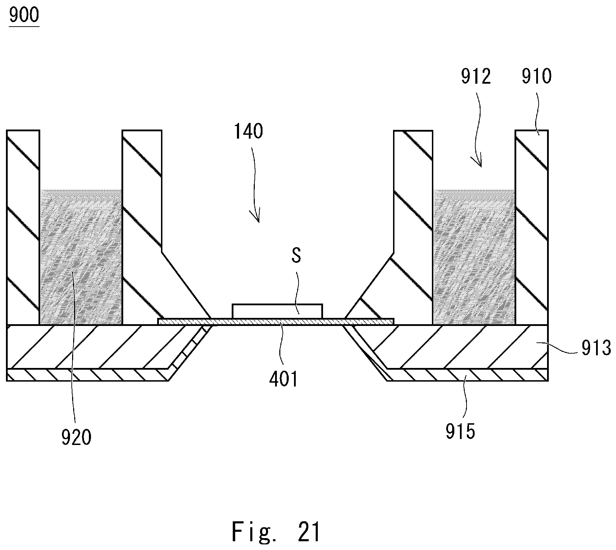

A modified example of a sample holder will now be described with reference to Fig. 21. Fig. 21 is a cross-sectional view schematically showing a configuration of a sample holder 900. In Fig. 21, components of the microscope have been omitted when appropriate. For example, the objective lens below the sample S and the like are omitted. Hereinafter, a configuration of the sample holder for maintaining the sample S at a low temperature will be described with reference to Fig. 21.

The sample holder 900 includes the substrate 401, an upper holder 910, a metal plate 913, and a lower holder 915. The sample S is placed on the substrate 401. As described earlier, a cryogen for freezing is supplied to the sample S.

The substrate 401 is held between the upper holder 910 and the lower holder 915. For example, a peripheral edge portion of the substrate 401 is arranged between the upper holder 910 and the lower holder 915. Furthermore, the upper holder 910 includes a cryogen storage tub 912 that stores a cryogen 920 for maintaining a low temperature. For example, the cryogen storage tub 912 stores the cryogen 920 that is liquid nitrogen or the like. The cryogen storage tub 912 is arranged outside the field of view 140. The upper holder 910 and the lower holder 915 are formed of heat insulating material. Accordingly, a temperature rise of the cryogen 920 can be suppressed.

Furthermore, the metal plate 913 is fixed to the lower holder 915. The metal plate 913 is arranged so as to come into contact with the cryogen 920. The metal plate 913 constitutes a bottom surface of the cryogen storage tub 912. The upper holder 910 is to be a side surface of the cryogen storage tub 912. In addition, a space formed by the upper holder 910 and the metal plate 913 constitutes the cryogen storage tub 912. The metal plate 913 is cooled by the cryogen 920. Therefore, the metal plate 913 is maintained at a low temperature.

The metal plate 913 is a heat-transfer member with high thermal conductivity. The metal plate 913 is in contact with the substrate 401. For example, the metal plate 913 extends to the outside from a peripheral edge portion of the substrate 401. The metal plate 913 cooled by the cryogen 920 maintains the substrate 401 and the sample S at a low temperature. Accordingly, a temperature rise of the sample S can be further suppressed. Accordingly, since a low temperature state can be maintained for a long period of time, the sample S that is frozen can be observed over a long period of time.

A temperature measurement mechanism using a thermocouple or the like may be provided in order to measure the temperature of the sample S. In addition, in order to remove the cryogen for freezing having been dripped to the sample S and prevent dew condensation, an atmosphere of inert gas such as nitrogen gas may be created around the sample S and the sample S may be observed. Accordingly, the sample S can be observed with the objective lens from either above or below the sample S.

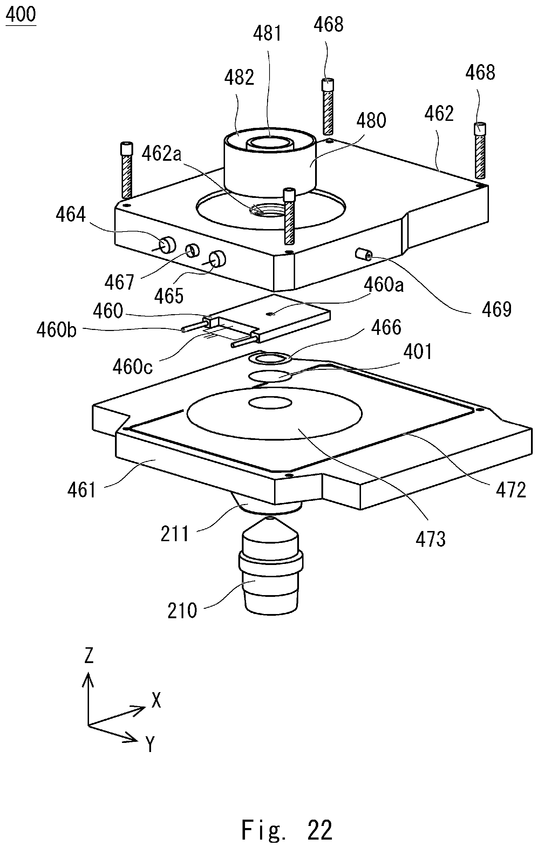

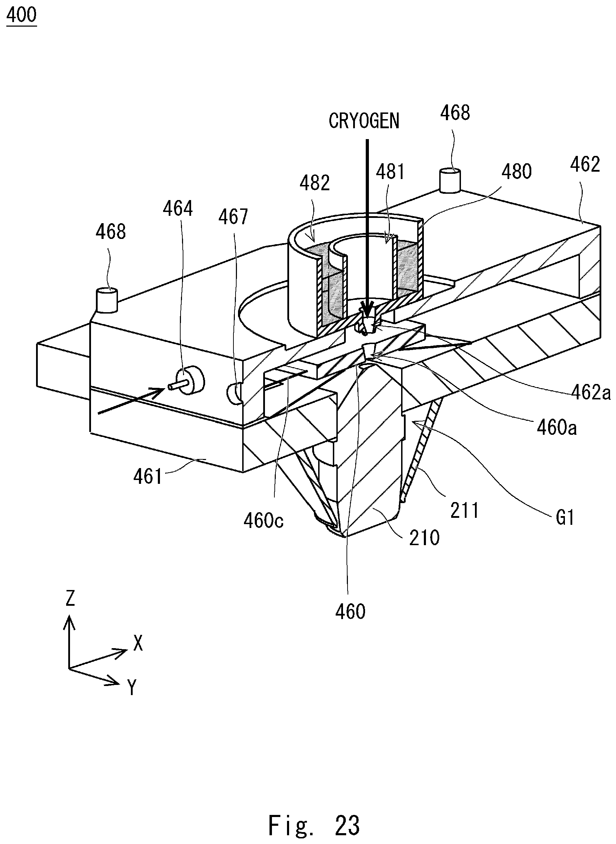

Next, a configuration of the sample holder 400 according to a modified example will be described with reference to Figs. 22 and 23. Fig. 22 is an exploded perspective view showing the configuration of the sample holder 400 including a cooling block. Fig. 23 is a perspective view showing a cross-sectional configuration of the sample holder 400. The sample holder 400 holds a cooling block 460 together with the substrate 401.

The sample holder 400 includes the lower holder 461 and the upper holder 462. The lower holder 461 and the upper holder 462 are fixed by a bolt 468. The cooling block 460 is attached to the upper holder 462. In addition, the substrate 401 and the cooling block 460 are arranged between the upper holder 462 and the lower holder 461. Furthermore, the lower holder 461 and the upper holder 462 may be fixed by connecting the lower holder 461 and the upper holder 462 with a hinge-like mechanism. Accordingly, opening and closing of the lower holder 461 and the upper holder 462 can be more readily performed.

The cooling block 460 is arranged above the substrate 401. In addition, a ring-shaped spacer 466 is provided between the cooling block 460 and the substrate 401. A sample stand 473 with an opening that supports the substrate 401 is provided below the substrate 401. In other words, the substrate 401 is pressed against the spacer 466 by the sample stand 473 with an opening and is arranged so as to face the cooling block 460 via the spacer 466. A sample containing liquid is arranged in a space enclosed by the spacer 466 on the substrate 401. The sample on the substrate 401 can be observed with the objective lens 210 through the opening portion of the sample stand 473 with an opening. A portion of the sample stand 473 with an opening that supports the substrate 401 may include a mechanism such as a spring.

In addition, the lower holder 461, the upper holder 462, the cooling block 460, the sample stand 473 with an opening, and the spacer 466 may include an opening for reducing fluid on the substrate 401 such as that described with reference to Figs. 18 to 20. Furthermore, a cooling tube for circulating liquid nitrogen, a temperature measurement mechanism using a thermocouple or the like, and a heater may be provided in the sample stand 473 with an opening to control the temperature of the space below the substrate 401. Accordingly, temperature stability of the sample on the substrate 401 can be improved. In addition, the sample stand 473 with an opening may include a circulation mechanism which supplies a solution such as an organic solvent that hardly freezes into a space between the substrate 401 and the objective lens 210 and which discharges the solution. Accordingly, the solution in an unfrozen state can be arranged in the space between the substrate 401 and the objective lens 210 and immersion objectives with high apertures can be used for observation.

A gas introduction port 469 for supplying an inert gas is provided on a side surface of the upper holder 462 in order to prevent dew condensation on the objective lens 210. The upper holder 462 includes a cryogen supply port 462a. The cryogen supply port 462a is, for example, a through-hole provided in the Z direction. Alternatively, the cryogen supply port 462a may be a screw hole for fixing a lid or an optical window to be described later. In addition, a cryogen introduction unit 480 is provided above the cryogen supply port 462a. The cryogen introduction unit 480 is a cylindrical member and includes a cryogen feeding port 481. A cryogen from the cryogen feeding port 481 is supplied to the sample through the cryogen supply port 462a. A liquid nitrogen storage tub 482 is formed around the cryogen feeding port 481. The liquid nitrogen storage tub 482 stores liquid nitrogen for precooling. Accordingly, a temperature rise is prevented by allowing the cryogen to come into contact with the cryogen introduction unit 480 before coming into contact with the sample.

The cooling block 460 is formed of a material with high thermal conductivity including a metal material such as aluminum, copper, or silver. The cooling block 460 is provided with a cooling tube 460b for liquid nitrogen to circulate. The cooling tube 460b serves as a flow path that passes inside the cooling block 460. A supply port 464 and a discharge port 465 of liquid nitrogen are provided on a side surface of the upper holder 462. The supply port 464 and the discharge port 465 are connected to the cooling tube 460b. The liquid nitrogen supplied from the supply port 464 circulates in the cooling block 460 and is discharged from the discharge port 465.

**In** addition, a thermocouple or a heater for temperature control is provided in the cooling block 460. A terminal 460c of the thermocouple and the heater is provided on a side surface of the cooling block 460. A port 467 for connection to a temperature sensor such as a thermocouple or a heater is provided on a side surface of the upper holder 462. A current is supplied to the heater from the port 467. **In** addition, a voltage signal of the thermocouple is retrieved from the port 467.

The cooling block 460 is provided with a through-hole 460a in the Z direction. The through-hole 460a is provided in the field of view of the objective lens 210. The through-hole 460a is positioned directly below the cryogen supply port 462a. The cryogen from the cryogen feeding port 481 is supplied to the sample through the through-hole 460a.

As described above, providing the cooling block 460 above the substrate 401 enables the sample to be maintained at a low temperature. For example, since the cooling block 460 comes into contact with the cryogen, the cryogen is maintained at a low temperature. The cooling block 460 is provided with a heater for temperature adjustment or a temperature sensor. Accordingly, the temperature of the sample can be controlled. A sample in a frozen state or a thawed sample can be easily observed.

Alternatively, after the sample is quickly frozen with a cryogen, the cryogen can be removed and the temperature of the sample can be controlled with only the cooling block 460. In this case, after removing the cryogen, a storage space is sealed by removing the cryogen introduction unit 480 and closing the cryogen supply port 462a with a lid or the like. By making the lid that closes the cryogen supply port 462a an optical window made of a transparent substrate such as glass, illumination light can be introduced from above the cryogen supply port 462a and the sample can be observed.

In the first to fourth embodiments, modified examples thereof, and the like described above, the sample to be observed may be adherent cells, floating cells, cell clusters, or the like or cell-derived material such as exosomes, RNA (Ribonucleic acid) or DNA (deoxyribonucleic acid). **In** addition, the material of the substrate that holds the sample may be water soluble. Accordingly, simply placing a substrate containing a sample such as frozen cells in a liquid such as a culture fluid dissolves the substrate, enables the sample to be separated from the substrate, and enables the sample to be used as-is for sample observation or culture.

**In** a holder that holds the sample according to the first to fourth embodiments, modified examples thereof, and the like described above, a surface of the holder in an area through which a cryogen passes may be coated with a highly insulating material or covered with a member made of a highly insulating material. Accordingly, a temperature rise of the cryogen being dripped and a resulting bumping can be prevented, thereby improving reproducibility of a sample freezing rate.

A part of or all of the embodiments described above may be described as, but not limited to, the following supplementary notes.

### (Supplementary Note 1)

A freezing apparatus, comprising:
a sample holder configured to hold a sample containing a liquid in a state where a volume of the liquid in a cryogen contact area differs from a volume of the liquid outside the cryogen contact area; and
sample freezing means for freezing the sample in the cryogen contact area with a cryogen.

### (Supplementary Note 2)

The freezing apparatus according to supplementary note 1, wherein
the sample holder is configured to hold the sample in a state where a height of a liquid level of the sample in the cryogen contact area is lower than outside the cryogen contact area.

### (Supplementary Note 3)

The freezing apparatus according to supplementary note 1 or 2, comprising an adjustment mechanism configured to adjust a height of a liquid level of the liquid.

### (Supplementary Note 4)

The freezing apparatus according to any one of supplementary notes 1 to 3, wherein
the sample holder is provided with a through-hole, and
the height of the liquid level of the liquid is adjusted by sucking the liquid from the through-hole.

### (Supplementary Note 5)

The freezing apparatus according to any one of supplementary notes 1 to 4, comprising:
in a space enclosed by the sample holder, a first supply port to which a chemical fixative for chemically fixing the sample is supplied; and
in the space, a second supply port to which a staining dye for staining the sample or a decolorizing agent for decolorizing the stained sample is supplied.

### (Supplementary Note 6)

The freezing apparatus according to any one of supplementary notes 1 to 5, wherein
the sample holder is provided with a sample retainer configured to press the sample, and
the sample retainer includes an opening portion that corresponds to the cryogen contact area.

### (Supplementary Note 7)

The freezing apparatus according to any one of supplementary notes 1 to 6, wherein
the height of the liquid level of the liquid is adjusted by pressing a pressing member provided above the sample against the sample.

### (Supplementary Note 8)

The freezing apparatus according to any one of supplementary notes 1 to 6, further comprising a low-temperature maintaining mechanism configured to maintain the cryogen at a low temperature after freezing the sample.

### (Supplementary Note 9)

The freezing apparatus according to any one of supplementary notes 1 to 8, further comprising a measurement mechanism configured to measure the height of the liquid level of the liquid.

### (Supplementary Note 10)

The freezing apparatus according to any one of supplementary notes 1 to 9, wherein
after freezing the sample, the sample is preserved in the frozen state.

### (Supplementary Note 11)

The freezing apparatus according to any one of supplementary notes 1 to 10, wherein
a sample preserved in the frozen state is thawed and cultured.

### (Supplementary Note 12)

The freezing apparatus according to any one of supplementary notes 1 to 11, comprising:
a cryogen container being arranged above a sample and including a release port for releasing a cryogen toward the sample;
an introduction path configured to introduce a cryogen or a material of the cryogen into the cryogen container; and
cooling means provided in the cryogen container for cooling the cryogen or the material of the cryogen.

### (Supplementary Note 13)

The freezing apparatus according to supplementary note 12, wherein
the cooling means includes a cooling machine or liquid nitrogen. (Supplementary Note 14)

The freezing apparatus according to supplementary note 12 or 13, wherein
a ring illumination light source configured to illuminate the sample is provided around the release port.

### (Supplementary Note 15)

The freezing apparatus according to any one of supplementary notes 1 to 14, wherein
the material of the cryogen is supplied to the cryogen container and the cryogen is manufactured in the cryogen container.

### (Supplementary Note 16)

The freezing apparatus according to any one of supplementary notes 1 to 15, wherein
a stirring machine configured to stir the material of the cryogen in order to generate the cryogen is attached to the cryogen container.

### (Supplementary Note 17)

The freezing apparatus according to any one of supplementary notes 1 to 16, wherein
an open/close valve is provided in the cryogen release port.

### (Supplementary Note 18)

The freezing apparatus according to supplementary note 17, wherein
opening/closing of the open/close valve is controlled by a signal from the sample or a signal for stimulating the sample.

### (Supplementary Note 19)

The freezing apparatus according to any one of supplementary notes 1 to 18, wherein
the sample is held on a substrate for sample freezing with a surface on which irregularities are formed.

### (Supplementary Note 20)

The freezing apparatus according to supplementary note 19, wherein
a depth of recessed portions of the irregularities ranges from 50 µm to 100 µm.

### (Supplementary Note 21)

The freezing apparatus according to supplementary note 19 or 20, wherein
a width of the recessed portions of the irregularities ranges from 100 µm to 300 µm.

### (Supplementary Note 22)

The freezing apparatus according to any one of supplementary notes 19 to 21, wherein
a reservoir configured to store a fluid is formed on the substrate for sample freezing.

### (Supplementary Note 23)

The freezing apparatus according to supplementary note 22, wherein
the reservoir is formed at an end portion in a first direction of the substrate for sample freezing in a top view, and
a suction port for sucking the liquid is formed at an end portion in a second direction that is orthogonal to the first direction of the sample holder.

### (Supplementary Note 24)

A microscope, comprising:
the freezing apparatus according to any one of supplementary notes 1 to 11; and
an objective lens configured to receive light from the sample, wherein
the cryogen contact area corresponds to a field of view of the objective lens, and
the sample freezing means is for freezing a sample in the field of view of the objective lens.

### (Supplementary Note 25)

The microscope according to supplementary note 24, further comprising:
a cooling block provided directly above the sample, wherein
a cooling tube configured to circulate a liquid for cooling the cooling block is provided inside the cooling block, and
a through-hole through which a cryogen for freezing the sample passes is provided.

### (Supplementary Note 26)

The microscope according to supplementary note 25, wherein
the cooling block is provided with at least one of a temperature sensor and a heater.

### (Supplementary Note 27)

A freezing method, comprising the steps of:
holding a sample containing a liquid with a sample holder in a state where a volume of the liquid of the sample in a cryogen contact area differs from a volume of the liquid outside the cryogen contact area; and
freezing the sample in the cryogen contact area with a cryogen.

### (Supplementary Note 28)

A freezing apparatus, comprising:
a cryogen container being arranged above a sample and including a release port for releasing a cryogen toward the sample;
an introduction path configured to introduce a cryogen or a material of the cryogen into the cryogen container; and
cooling means provided in the cryogen container for cooling the cryogen or the material of the cryogen.

### (Supplementary Note 29)

The freezing apparatus according to supplementary note 28, wherein
the cooling means includes a cooling machine or liquid nitrogen. (Supplementary Note 30)

The freezing apparatus according to supplementary note 28 or 29, wherein
a ring illumination light source configured to illuminate the sample is provided around the release port.

### (Supplementary Note 31)

The freezing apparatus according to any one of supplementary notes 28 to 30, wherein
the material of the cryogen is supplied to the cryogen container and the cryogen is manufactured in the cryogen container.

### (Supplementary Note 32)

The freezing apparatus according to any one of supplementary notes 28 to 31, wherein
a stirring machine configured to stir the material of the cryogen in order to generate the cryogen is attached to the cryogen container.

### (Supplementary Note 33)

The freezing apparatus according to any one of supplementary notes 28 to 32, wherein
an open/close valve is provided in the release port.

### (Supplementary Note 34)

The freezing apparatus according to supplementary note 33, wherein
opening/closing of the open/close valve is controlled by a signal from the sample or a signal for stimulating the sample.

### (Supplementary Note 35)

The freezing apparatus according to any one of supplementary notes 28 to 34, wherein
the sample is held on a substrate for sample freezing with a surface on which irregularities are formed.

### (Supplementary Note 36)

The freezing apparatus according to supplementary note 35, wherein
a depth of recessed portions of the irregularities ranges from 50 µm to 100 µm.

### (Supplementary Note 37)

The freezing apparatus according to supplementary note 35 or 36, wherein
a width of the recessed portions of the irregularities ranges from 100 µm to 300 µm.

### (Supplementary Note 38)

The freezing apparatus according to any one of supplementary notes 35 to 37, wherein
a reservoir configured to store a liquid is formed on the substrate for sample freezing.

### (Supplementary Note 39)

The freezing apparatus according to supplementary note 38, wherein
the reservoir is formed at an end portion in a first direction of the substrate for sample freezing in a top view, and
a suction port for sucking the liquid is formed at an end portion in a second direction that is orthogonal to the first direction of the sample holder.

### (Supplementary Note 40)

A microscope, comprising:
the freezing apparatus according to any one of supplementary notes 28 to 39;
a sample holder that is provided so as to enclose a sample and that includes an opening portion to which the cryogen released from the release port is supplied;
a stage on which the sample holder is placed; and
an objective lens arranged below the stage in order to observe the sample frozen with the cryogen.

### (Supplementary Note 41)

The microscope according to supplementary note 40, further comprising a temperature adjustment mechanism configured to adjust a temperature of the sample.

### (Supplementary Note 42)

The microscope according to supplementary note 40 or 41, wherein
the sample holder includes:
a first supply port configured to supply a chemical fixative for chemically fixing the sample to a space enclosed by the sample holder; and
a second supply port configured to supply a staining dye for staining the sample or a decolorizing agent for decolorizing the stained sample to the space.

### (Supplementary Note 43)

The microscope according to any one of supplementary notes 40 to 42, further comprising:
a cooling block arranged directly above the sample, wherein
a cooling tube in which a cooling liquid for cooling the sample circulates is provided in the cooling block, and
the cooling block is provided with a through-hole through which a cryogen from the freezing apparatus passes.

### (Supplementary Note 44)

The microscope according to supplementary note 43, wherein
the cooling block is provided with a temperature sensor.

### (Supplementary Note 45)

The microscope according to supplementary note 44, wherein
the cooling block is provided with a heater configured to heat a sample.

### (Supplementary Note 46)

An observation method, comprising the steps of:
(1) supplying a cryogen to a sample in a field of view of a microscope and freezing the sample;
(2) supplying a chemical fixative to the frozen sample and chemically fixing the sample;
(3) supplying a staining dye to the chemically fixed sample and staining the sample; and
(4) observing the stained sample with the microscope.

### (Supplementary Note 47)

The observation method according to supplementary note 46, comprising the steps of:
(5) supplying a decolorizing agent to the sample in the field of view after observation; and
(6) supplying a different staining dye to the decolorized sample and staining the sample.

### (Supplementary Note 48)

A freezing method, comprising the steps of:
arranging a sample of a fluid containing cells in a plurality of recessed portions provided on a substrate for sample freezing; and
supplying a cryogen in order to freeze the sample arranged in the recessed portions.

### (Supplementary Note 49)

The freezing method according to supplementary note 48, wherein
a depth of the recessed portions ranges from 50 µm to 100 µm.

### (Supplementary Note 50)

The freezing method according to supplementary note 48 or 49, wherein
a width of the recessed portions ranges from 100 µm to 300 µm.

### (Supplementary Note 51)

The freezing method according to any one of supplementary notes 48 to 50, wherein
the sample is frozen by bringing a cryogen into contact with the sample in a state where the substrate for sample freezing is in a field of view of a microscope.

### (Supplementary Note 52)

The freezing method according to any one of supplementary notes 48 to 51, wherein
a reservoir configured to store a fluid is formed on the substrate for sample freezing.

### (Supplementary Note 53)

A substrate for sample freezing for freezing a sample using a cryogen, wherein
irregularities for holding a sample of a fluid containing cells are formed on a surface of the substrate for sample freezing.

### (Supplementary Note 54)

The substrate for sample freezing according to supplementary note 53, wherein
a depth of recessed portions of the irregularities ranges from 50 µm to 100 µm.

### (Supplementary Note 55)

The substrate for sample freezing according to supplementary note 53 or 54, wherein
a width of the recessed portions of the irregularities ranges from 100 µm to 300 µm.

### (Supplementary Note 56)

The substrate for sample freezing according to any one of supplementary notes 53 to 55, wherein
a reservoir configured to store a fluid is formed on the substrate for sample freezing.

### (Supplementary Note 57)

A freezing apparatus, comprising:
the substrate for sample freezing according to any one of supplementary notes 53 to 55; and
a cryogen container being arranged above the substrate for sample freezing and including a release port for releasing a cryogen toward the sample.

### (Supplementary Note 58)

The freezing apparatus according to supplementary note 57, wherein
the sample is thawed using a heat source.

### (Supplementary Note 59)

A microscope, comprising:
an objective lens;
the substrate for sample freezing according to any one of supplementary notes 53 to 55 arranged in a field of view of the objective lens; and
a cryogen container being arranged above the substrate for sample freezing and including a release port for releasing a cryogen toward the sample.

### (Supplementary Note 60)

The microscope according to supplementary note 59, wherein
the sample is thawed using a heat source.

### (Supplementary Note 61)

The microscope according to any one of supplementary notes 59 to 60, wherein
the microscope holds a cooling block arranged directly above the sample,
the cooling block is provided with a through-hole through which the cryogen passes, and
the cooling block is provided with a heater for temperature adjustment and a temperature sensor.

### (Supplementary Note 62)

A freezing apparatus, comprising:
a sample substrate configured to hold a sample containing a liquid;
a suction port connected to a space above the sample substrate in order to suck the liquid; and
cryogen supplying means for supplying a cryogen with respect to the sample after the liquid is sucked from the suction port.

### (Supplementary Note 63)

A freezing apparatus, comprising:
a sample holder configured to hold a sample containing a liquid; and
sample freezing means for freezing the sample with a cryogen, wherein
a height of the liquid is adjusted by sucking the liquid from a through-hole provided in the sample holder.

### (Supplementary Note 64)

The freezing apparatus according to supplementary note 63, wherein
the sample holder includes a supply port for supplying a fluid with respect to the sample.

### (Supplementary Note 65)

The freezing apparatus according to supplementary note 63 or 64, wherein
the sample freezing means includes a cryogen container being arranged above the sample and including a release port for releasing a cryogen toward the sample.

### (Supplementary Note 66)

The freezing apparatus according to supplementary note 65, wherein
the sample freezing means includes:
an introduction path configured to introduce a cryogen or a material of the cryogen into the cryogen container; and
cooling means provided in the cryogen container for cooling the cryogen or the material of the cryogen.

### (Supplementary Note 67)

The freezing apparatus according to any one of supplementary notes 63 to 66, wherein
the sample holder is configured to hold a substrate for sample freezing with a surface on which irregularities for holding a sample of a fluid containing cells are formed.

### (Supplementary Note 68)

The freezing apparatus according to supplementary note 67, wherein
a depth of recessed portions of the irregularities ranges from 50 µm to 100 µm.

### (Supplementary Note 69)

The freezing apparatus according to supplementary note 67 or 68, wherein
a width of the recessed portions of the irregularities ranges from 100 µm to 300 µm.

### (Supplementary Note 70)

The freezing apparatus according to any one of supplementary notes 63 to 69, wherein
the sample holder is configured to hold the sample in a state where a height of a liquid level of the sample in the cryogen contact area is lower than outside the cryogen contact area.

### (Supplementary Note 71)

The freezing apparatus according to supplementary note 70, further comprising:
a cooling block provided directly above the sample, wherein
a cooling tube configured to circulate a liquid for cooling the cooling block is provided inside the cooling block, and
a through-hole through which a cryogen for freezing the sample passes is provided with the cooling block.

### (Supplementary Note 72)

The freezing apparatus according to supplementary note 71, wherein
the cooling block is provided with a temperature sensor.

### (Supplementary Note 73)

The freezing apparatus according to supplementary note 72, wherein
the cooling block is provided with a heater configured to heat a sample.

### (Supplementary Note 74)

A microscope, comprising:
the freezing apparatus according to any one of supplementary notes 62 to 73; and
an objective lens configured to receive light from the sample.

### (Supplementary Note 75)

A microscope, comprising:
an objective lens configured to receive light from a sample;
sample freezing means for freezing a sample by supplying a cryogen to the sample in a field of view of the objective lens; and
a cooling block being arranged directly above the sample in order to cool the sample or the cryogen and including a through-hole through which the cryogen passes.

### (Supplementary Note 76)

The microscope according to supplementary note 75, wherein
a cooling tube configured to circulate a liquid for cooling the cooling block is provided inside the cooling block.

### (Supplementary Note 77)

The microscope according to supplementary note 75 or 76, wherein
the cooling block is provided with a temperature sensor.

### (Supplementary Note 78)

The microscope according to any one of supplementary notes 75 to 77, wherein
the cooling block is provided with a heater configured to heat a sample.

### (Supplementary Note 79)

An observation method, comprising the steps of:
freezing the sample in a field of view of an objective lens using the method according to supplementary note 27, 49, 50, 51, or 52; and
observing the frozen sample in the cryogen contact area using the objective lens.

### (Supplementary Note 80)

The observation method according to supplementary note 79, wherein
the sample is observed while the sample is being frozen with the cryogen.

Any two or more of the first to fourth embodiments can be used in combination as appropriate. As described above, while the invention made by the present inventors has been specifically explained based on the embodiments, it goes without saying that the present invention is not limited to the embodiments described above and can be modified in various ways without departing from the gist thereof.

The present application claims priority on the basis of Japanese Patent Application No. 2022-189880 filed on November 29, 2022 and Japanese Patent Application No. 2023-122175 filed on July 27, 2023, the entire contents of which are incorporated herein by reference.

### Reference Signs List

- 10: MICROSCOPE
- 12: MICROSCOPE MAIN BODY
- 110: FIRST PLATE
- 111: CRYOGEN STORAGE TUB
- 112: CYLINDRICAL PORTION
- 114: THROUGH-HOLE
- 120: SECOND PLATE
- 124: THROUGH-HOLE
- 129: STORAGE SPACE
- 130: RUBBER
- 140: FIELD OF VIEW
- 201: LIGHT SOURCE
- 202: LENS
- 203: BEAM SPLITTER
- 204: IMAGING LENS
- 205: PHOTODETECTOR
- 210: OBJECTIVE LENS
- 300: SYRINGE
- 301: MICROMETER
- 310: PIPING TUBE
- 400: SAMPLE HOLDER
- 401: SUBSTRATE
- 403: WALL
- 405: SPACER
- 411: BASE
- 412: SAMPLE RETAINER
- 412a: OPENING PORTION
- 421: SUPPLY PIPE
- 422: SUPPLY PIPE
- 423: SUPPLY PIPE
- 431: DISCHARGE PIPE
- 432: DISCHARGE PIPE
- 450: INERT GAS
- 460: COOLING BLOCK
- 460a: THROUGH-HOLE
- 460b: COOLING TUBE
- 460c: TERMINAL
- 461: LOWER HOLDER
- 462: UPPER HOLDER
- 462a: CRYOGEN SUPPLY PORT
- 463: SUCTION PORT
- 465: DISCHARGE PORT
- 467: PORT
- 468: BOLT
- 469: GAS INTRODUCTION PORT
- 472: SEALING MEMBER
- 473: SAMPLE STAND WITH OPENING
- 480: CRYOGEN INTRODUCTION UNIT
- 481: CRYOGEN FEEDING PORT
- 482: LIQUID NITROGEN STORAGE TUB
- 500: CRYOGEN FEEDING APPARATUS
- 501: CRYOGEN
- 503: METAL MEMBER
- 600: MICROSCOPE
- 601: OBSERVATION LIGHT SOURCE
- 602: LENS
- 603: DICHROIC MIRROR
- 604: DICHROIC MIRROR
- 605: OBJECTIVE LENS
- 610: STAGE
- 620: OBSERVATION OPTICAL SYSTEM
- 621: FILTER
- 622: TUBE LENS
- 623: TWO-DIMENSIONAL PHOTODETECTOR
- 630: THAWING LIGHT SOURCE
- 640: THAWING OPTICAL SYSTEM
- 641: SHUTTER
- 642: SCANNER
- 700: CRYOGEN FEEDING APPARATUS
- 701: CRYOGEN
- 702: INTRODUCTION PATH
- 703: COOLING MEANS
- 705: CRYOGEN CONTAINER
- 710: RELEASE PORT
- 711: STIRRING MECHANISM
- 713: OPEN/CLOSE VALVE
- 714: ILLUMINATION LIGHT SOURCE
- 730: LID
- 750: HOLDER
- 800: SAMPLE SUBSTRATE
- 801: GROOVE
- 804: PROJECTING PORTION
- 805: RECESSED PORTION
- 807: RESERVOIR
- 808: GROOVE
- 900: SAMPLE HOLDER
- 910: UPPER HOLDER
- 912: CRYOGEN STORAGE TUB
- 913: METAL PLATE
- 915: LOWER HOLDER
- 920: CRYOGEN
- S: SAMPLE
- S1: LIQUID
- S2: BIOLOGICAL SAMPLE
- S5: FLUID
- S6: CELL
- S7: FROZEN SAMPLE
- G1: OBJECTIVE LENS SPACE
- G2: SAMPLE SPACE

## Claims

1. A freezing apparatus, comprising:
a sample holder configured to hold a sample containing a liquid in a state where a volume of the liquid in a cryogen contact area differs from a volume of the liquid outside the cryogen contact area; and
sample freezing means for freezing the sample in the cryogen contact area with a cryogen.

2. The freezing apparatus according to claim 1, wherein
the sample holder is configured to hold the sample in a state where a height of a liquid level of the sample in the cryogen contact area is lower than outside the cryogen contact area.

3. The freezing apparatus according to claim 1 or 2, comprising an adjustment mechanism configured to adjust a height of a liquid level of the liquid.

4. The freezing apparatus according to any one of claims 1 to 3,
wherein
the sample holder is provided with a through-hole, and
the height of the liquid level of the liquid is adjusted by sucking the liquid from the through-hole.

5. The freezing apparatus according to any one of claims 1 to 4,
comprising:
a first supply port configured to supply a chemical fixative for chemically fixing the sample to a space enclosed by the sample holder; and
a second supply port configured to supply a staining dye for staining the sample or a decolorizing agent for decolorizing the stained sample to the space.

6. The freezing apparatus according to any one of claims 1 to 5,
wherein
the sample holder is provided with a sample retainer configured to press the sample, and
the sample retainer includes an opening portion that corresponds to the cryogen contact area.

7. The freezing apparatus according to any one of claims 1 to 6, wherein
the height of the liquid level of the liquid is adjusted by pressing a pressing member provided above the sample against the sample.

8. The freezing apparatus according to any one of claims 1 to 7, further comprising a low-temperature maintaining mechanism configured to maintain the cryogen at a low temperature after freezing the sample.

9. The freezing apparatus according to any one of claims 1 to 8, further comprising a measurement mechanism configured to measure the height of the liquid level of the liquid.

10. The freezing apparatus according to any one of claims 1 to 9, wherein
after freezing the sample, the sample is preserved in the frozen state.

11. The freezing apparatus according to any one of claims 1 to 10, wherein
a sample preserved in the frozen state is thawed and cultured.

12. A microscope, comprising:
the freezing apparatus according to any one of claims 1 to 11; and
an objective lens configured to receive light from the sample, wherein
the cryogen contact area corresponds to a field of view of the objective lens, and
the sample freezing means is for freezing a sample in the field of view of the objective lens.

13. The microscope according to claim 12, further comprising:
a cooling block provided directly above the sample, wherein
a cooling tube configured to circulate a liquid for cooling the cooling block is provided inside the cooling block,
a through-hole through which a cryogen for freezing the sample passes is provided, and
the cooling block is provided with at least one of a temperature sensor and a heater.

14. A freezing method, comprising the steps of:
holding a sample containing a liquid with a sample holder in a state where a volume of the liquid of the sample in a cryogen contact area differs from a volume of the liquid outside the cryogen contact area; and
freezing the sample in the cryogen contact area with a cryogen.

15. An observation method, comprising the steps of:
freezing the sample in a field of view of an objective lens using the freezing method according to claim 14; and
observing the frozen sample in the cryogen contact area using the objective lens.
